# EUROPEAN PATENT SPECIFICATION

(11) **EP 2 850 433 B1**
(45) Date of publication and mention of the grant of the patent: **15.08.2018**
(21) Application number: 13739819.4
(22) Date of filing: 16.05.2013
(51) Int. Cl.: G01N 33/574, A61P 35/00, G01N 33/50

(54) **USE OF TROP-2 AS PREDICTIVE MARKER OF RESPONSE TO ANTI-TUMOR THERAPY BASED ON INHIBITORS OF AKT**
VERWENDUNG VON TROP-2 ALS PRÄDIKTIVER MARKER DER REAKTION AUF EINE ANTITUMORTHERAPIE AUF GRUNDLAGE VON AKT-HEMMERN
UTILISATION DE TROP-2 EN TANT QUE MARQUEUR PRÉDICTIF DE RÉPONSE À UNE THÉRAPIE ANTICANCÉREUSE À BASE D'INHIBITEURS D'AKT

(30) Priority: 16.05.2012 IT CH20120008
(43) Date of publication of application: 25.03.2015
(73) Proprietor: ONCOXX Biotech s.r.l., 66034 Lanciano (IT)
(72) Inventor: GUERRA, Emanuela, 66100 Chieti (IT) (IT); ALBERTI, Saverio, 66100 Chieti (IT) (IT)
(74) Representative: Statti, Francesco
(86) International application number: PCT/IT2013/000139
(87) International publication number: WO 2013/171777

(56) References cited:
- WO-A1-2011/130654
- WO-A2-2011/085276
- CA-A1- 2 798 778
- JOYCE VARUGHESE ET AL: "Uterine serous papillary carcinomas overexpress human trophoblast-cell-surface marker (trop-2) and are highly sensitive to immunotherapy with hRS7, a humanized anti-trop-2 monoclonal antibody", CANCER, vol. 117, no. 14, 15 July 2011 (2011-07-15), pages 3163-3172, XP55057457, ISSN: 0008-543X, DOI: 10.1002/cncr.25891
- JOYCE VARUGHESE ET AL: "Cervical carcinomas overexpress human trophoblast cell-surface marker (Trop-2) and are highly sensitive to immunotherapy with hRS7, a humanized monoclonal anti-Trop-2 antibody", AMERICAN JOURNAL OF OBSTETRICS & GYNECOLOGY, MOSBY, ST LOUIS, MO, US, vol. 205, no. 6, 29 June 2011 (2011-06-29) , pages 567.e1-567.e7, XP028116550, ISSN: 0002-9378, DOI: 10.1016/J.AJOG.2011.06.093 [retrieved on 2011-07-13]
- JOYCE VARUGHESE ET AL: "High-grade, chemotherapy-resistant primary ovarian carcinoma cell lines overexpress human trophoblast cell-surface marker (Trop-2) and are highly sensitive to immunotherapy with hRS7, a humanized monoclonal anti-Trop-2 antibody", GYNECOLOGIC ONCOLOGY, ACADEMIC PRESS, LONDON, GB, vol. 122, no. 1, 3 March 2011 (2011-03-03) , pages 171-177, XP028221428, ISSN: 0090-8258, DOI: 10.1016/J.YGYNO.2011.03.002 [retrieved on 2011-03-10]
- RHODA RAJI ET AL: "Uterine and ovarian carcinosarcomas overexpressing Trop-2 are sensitive to hRS7, a humanized anti-Trop-2 antibody", JOURNAL OF EXPERIMENTAL & CLINICAL CANCER RESEARCH, vol. 30, no. 1, 1 January 2011 (2011-01-01), page 106, XP55052404, ISSN: 1756-9966, DOI: 10.1158/0008-5472.CAN-07-6135
- M TREROTOLA ET AL: "Upregulation of Trop-2 quantitatively stimulates human cancer growth", ONCOGENE, vol. 32, no. 2, 20 February 2011 (2011-02-20), pages 222-233, XP55057442, ISSN: 0950-9232, DOI: 10.1038/onc.2012.36
- ELIANA BIGNOTTI ET AL: "Trop-2 protein overexpression is an independent marker for predicting disease recurrence in endometrioid endometrial carcinoma", BMC CLINICAL PATHOLOGY, vol. 12, no. 1, 1 January 2012 (2012-01-01), page 22, XP55255567, GB ISSN: 1472-6890, DOI: 10.1186/1756-0500-5-65
- JAU-CHEN LIN ET AL: "TROP2 is epigenetically inactivated and modulates IGF-1R signalling in lung adenocarcinoma", EMBO MOLECULAR MEDICINE, vol. 4, no. 6, 15 March 2012 (2012-03-15), pages 472-485, XP55255417, Weinheim ISSN: 1757-4676, DOI: 10.1002/emmm.201200222
- VARUGHESE JOYCE ET AL: "Uterine serous papillary carcinomas overexpress human trophoblast-cell-surface marker (Trop-2) and are highly sensitive to immunotherapy with hRS7, a humanized anti-Trop-2 monoclonal antibody.", CANCER 15 JUL 2011, vol. 117, no. 14, 15 July 2011 (2011-07-15), pages 3163-3172, XP055057457, ISSN: 1097-0142
- VARUGHESE JOYCE ET AL: "Cervical carcinomas overexpress human trophoblast cell-surface marker (Trop-2) and are highly sensitive to immunotherapy with hRS7, a humanized monoclonal anti-Trop-2 antibody.", AMERICAN JOURNAL OF OBSTETRICS AND GYNECOLOGY DEC 2011, vol. 205, no. 6, December 2011 (2011-12), pages 567.e1-7, XP028116550, ISSN: 1097-6868
- VARUGHESE JOYCE ET AL: "High-grade, chemotherapy-resistant primary ovarian carcinoma cell lines overexpress human trophoblast cell-surface marker (Trop-2) and are highly sensitive to immunotherapy with hRS7, a humanized monoclonal anti-Trop-2 antibody.", GYNECOLOGIC ONCOLOGY JUL 2011, vol. 122, no. 1, July 2011 (2011-07), pages 171-177, XP028221428, ISSN: 1095-6859
- RAJI RHODA ET AL: "Uterine and ovarian carcinosarcomas overexpressing Trop-2 are sensitive to hRS7, a humanized anti-Trop-2 antibody.", JOURNAL OF EXPERIMENTAL & CLINICAL CANCER RESEARCH : CR 2011, vol. 30, 2011, page 106, XP055052404, ISSN: 1756-9966
- TREROTOLA M ET AL: "Upregulation of Trop-2 quantitatively stimulates human cancer growth.", ONCOGENE 10 JAN 2013, vol. 32, no. 2, 20 February 2012 (2012-02-20), pages 222-233, XP055057442, ISSN: 1476-5594
- NAKASHIMA KAZUE ET AL: "Serological identification of TROP2 by recombinant cDNA expression cloning using sera of patients with esophageal squamous cell carcinoma.", INTERNATIONAL JOURNAL OF CANCER. JOURNAL INTERNATIONAL DU CANCER 20 DEC 2004, vol. 112, no. 6, 20 December 2004 (2004-12-20), pages 1029-1035, XP002694347, ISSN: 0020-7136
- HUAYI HUANG ET AL: "Aberrant expression of novel and previously described cell membrane markers in human breast cancer cell lines and tumors", CLINICAL CANCER RESEARCH, THE AMERICAN ASSOCIATION FOR CANCER RESEARCH, US, vol. 11, no. 12, 15 June 2005 (2005-06-15) , pages 4357-4364, XP008128315, ISSN: 1078-0432, DOI: 10.1158/1078-0432.CCR-04-2107
- ELIANA BIGNOTTI ET AL: "Trop-2 protein overexpression is an independent marker for predicting disease recurrence in endometrioid endometrial carcinoma", BMC CLINICAL PATHOLOGY, vol. 12, no. 1, 1 January 2012 (2012-01-01), page 22, XP055255567, GB ISSN: 1472-6890, DOI: 10.1186/1756-0500-5-65
- JAU-CHEN LIN ET AL: "TROP2 is epigenetically inactivated and modulates IGF-1R signalling in lung adenocarcinoma", EMBO MOLECULAR MEDICINE, vol. 4, no. 6, 15 March 2012 (2012-03-15), pages 472-485, XP055255417, Weinheim ISSN: 1757-4676, DOI: 10.1002/emmm.201200222

## Description

### FIELD

The present invention relates to anticancer therapy in patients bearing tumors overexpressing Trop-2, by means of drugs directed against a component of the the signalling network of Trop-2, wherein said component is Akt. Trop-2 overexpression can also be exploited for the screening in vitro and in vivo of the anticancer activity of new compounds.

### STATE OF THE ART

The latest generation of targeted anti-cancer therapies make use of tyrosine-kinase inhibitors (TKIs) and / or monoclonal antibodies (mAbs), having dominant oncogenic kinases and / or growth factors receptors as their targets. However, the clinical benefit associated with these agents is generally restricted to subsets of treated patients, in many cases defined by specific molecular changes (mutations, amplifications / deletions, increased / decreased expression) in the tumor. This finding has highlighted the possible importance of tumor genotypes / phenotypes as determinants of sensitivity to specific treatments and the resulting stratification of patients before treatment on the basis of molecular alterations used as biomarkers. These biomarkers are studied in order to be used as "predictive markers", ie able to predict the response or the resistance to a targeted therapy, with the aim of optimizing the clinical results through an effective personalization of treatment.

Predictive biomarkers are currently used in the clinic as a guide for the choice of very few targeted treatments. Examples include receptors for estrogen (ER) and progesterone, which are used to select patients with breast cancer to be treated with endocrine therapy; amplification of HER-2 for treatment with Trastuzumab; BCR-ABL translocation in chronic myeloid leukemia and mutations of c-kit in gastrointestinal stromal tumors for treatment with Imatinib; EGFR mutations in lung cancer for treatment with Gefitinib / Erlotinib ; KRAS mutations in colo-rectal cancer for treatment with Cetuximab.

"Enrichment biomarkers", i.e. markers that can predict which subgroups of patients may respond to treatment are a vital necessity. In fact they can be used to focus the experimentation towards subgroups in which it could be useful, increasing the likelihood of success and the effectiveness of the trials of new drugs. The combination HER2/Herceptest/Trastuzumab provides an example of successful use of diagnostic tools for targeted therapy. Other cases, although not many, of predictive and enrichment markers support the usefulness of this strategy.

However, in most cases, the currently available biological markers refer to either the therapeutic target itself or an individual component of the main signalling pathway through which the target is believed to act. The common limitation to these strategies is to consider the alteration of a single signalling pathway as the only alteration responsible for the growth of a tumor. At the basis of tumorigenesis, instead, there is a process of multi-stage progression. Multiple lines of evidence, including data obtained from the sequencing of entire exomes and microarray analyses of gene expression profiles of tumor reveal an extremely large number of different mutations and a great complexity of interactions between different signalling pathways in tumors. These investigations classify tumors into differentiated, clinically-relevant subgroups and highlight the importance of hitting multiple signaling pathways or final common pathways in order to obtain an effective treatment.

Genetic alterations which are classically involved in tumorigenesis concern oncogenes, such as PI3K, Ras, BCR-ABL, EGFR, HER-2 ("oncogene addiction"), and are necessary both for triggering and for maintaining the oncogenic condition, and are therefore a logical target for therapy. However, the tumorigenic state also depends on a wide variety of genes and pathways involved in normal cell functions, which present an altered regulation, although they are not intrinsically oncogenic ("non-oncogene addiction"). This addiction can provide a large number of drug targets with differential toxicity between normal and tumor cells: among them, some are being studied on pre-clinical models. Only some of these targets are being tested in clinical trials or are already in use for therapy (VEGF, for example, for treatment with Bevacizumab or Sorafenib / Sunitinib.

Therefore, in order to improve the effectiveness of existing therapies and to identify new therapeutic targets, the analysis of classical oncogenes must be accompanied the study of normal signalling pathways. These "normal" markers, however, while emerging as important factors in supporting tumorigenesis are still little used for the prediction of response to therapy in man.

Trop-2 (AC: P09758) is a transmembrane glycoprotein able to transduce a cytoplasmic calcium increase signal, is involved in cell-cell adhesion in epithelial tissues, and drives signalling networks that induce tumor growth (Guerra, Trerotola et al. 2013). Trop-2 has structural characteristics which are different from those of the four classical families of adhesion molecules, i.e. integrins, cadherins, selectins, and Ig-CAM. The extracellular domain of Trop-2 contains a cysteine-rich globular portion with an EGF-like domain (GA733 type-I domain (Chong and Speicher 2001)) and a thyroglobulin domain (Linnenbach, Seng et al. 1993; Fornaro, Dell'Arciprete et al. 1995; El Sewedy, Fornaro et al. 1998), which are necessary for the formation of homo-multimers (Balzar, Briaire-de Bruijn et al. 2001). A region without cysteines ("stem") connects the globular portion of Trop-2 to the hydrophobic transmembrane helix. The intracellular portion of Trop-2 consists of a tail of 26 aa devoid of enzymatic activity, containing a phosphoinositide-binding HIKE (El Sewedy, Fornaro et al. 1998; Ciccarelli, Acciarito et al. 2000). HIKE is found in signal- transducer molecules and can act as a docking site for effector /regulatory molecules / such as G-proteins, PKC, calmodulin (Alberti 1999). HIKE also includes a PKC phosphorylation site (S303) (Basu, Goldenberg et al. 1995).

Trop-2 expression has been associated with tumor growth in experimental models (Klein, Hartmann et al. 1990; Basu, Goldenberg et al. 1995; Wang, Day et al. 2008; Cubas, Zhang et al. 2010; Goldstein, Stoyanova et al. 2010). In human tumors, overexpression of Trop-2 is a negative prognostic factor in tumors of the pancreas, stomach, oral cavity, ovary, lung and colo-rectal cancer (Trerotola, Cantanelli et al. 2013). Moreover Trop-2 is also a marker of progenitor cells in prostate cancer (Goldstein, Stoyanova et al. 2010; Trerotola, Rathore et al. 2010) .

The Authors of the present invention have previously demonstrated that Trop-2 is expressed at high levels by the majority of tumors and tumor cell lines in man (Trerotola, Cantanelli et al. 2013). In particular, the Authors demonstrated that Trop-2 is overexpressed in cancer cells even when compared to tissues of origin that already express it (S. Alberti "Anti-Trop-2 monoclonal antibodies and uses thereof in the treatment and diagnosis of tumors" - PCT/IT2009/000035), indicating that the increased expression confers a selective advantage to tumor cells (Trerotola, Cantanelli et al. 2013).

The Authors of the present invention have previously demonstrated the function of stimulus of Trop-2 in the development of tumors,both in vitro and in vivo. Overexpression of Trop-2 stimulates the growth of both immortalized and tumor cells, with an increase in the proportion of cells in the S phase of the cell cycle. On the other hand, the use of small-interfering (si) RNAs directed against Trop-2 abolishes the proliferation of breast cancer and colon cancer cells lines which express Trop-2. Similar results have also been obtained in experimental tumors: the expression of Trop-2 increases tumor growth dramatically, and this growth is directly proportional to the levels of expression.

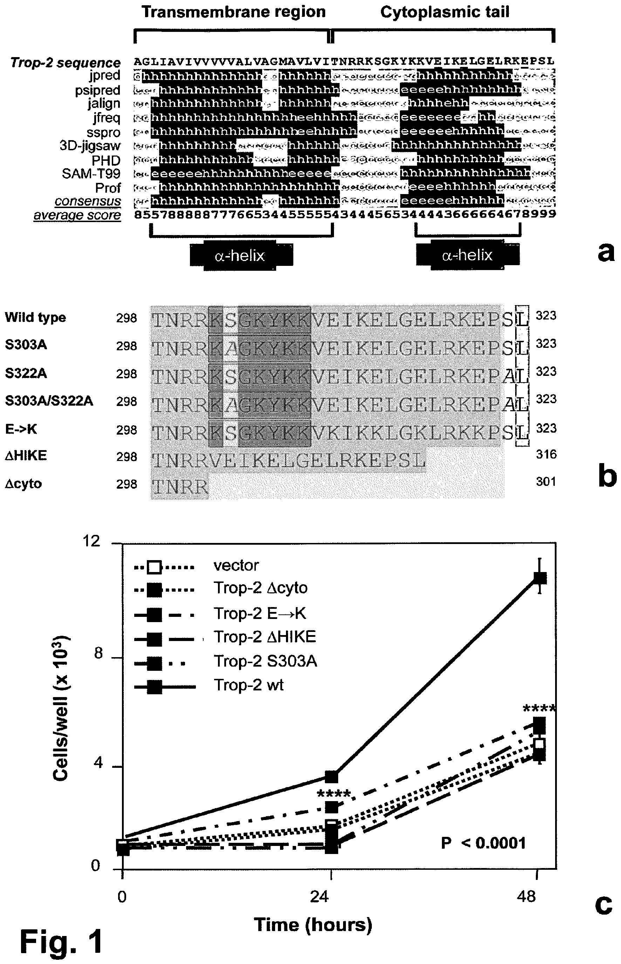

The integrity of the cytoplasmic tail of Trop-2 is necessary in order to have the growth stimulus: deletion of the entire tail or the HIKE domain, point mutations of serine 303 (phosphorylated by da PKCα) and 322, replacement of the four glutamic acid residues, which confer a negative charge all along one side of the α-helix, with lysine positive residues, all cause loss of Trop-2 stimulatory activity (Figure 1).

The stimulation of cell growth, both in vitro and in vivo, has been obtained through the overexpression of a normal Trop-2. Consistent with this, the Authors of the present invention have not identified mutations or structural alterations of the TROP2 gene in tumors, either in the coding region and in the 5 'and 3' untranslated regions (UTR), or in the regions proximal to the promoter (Trerotola, Cantanelli et al. 2013).

These results indicate that an increase in Trop-2 expression, in the absence of mutations, is both necessary and sufficient to stimulate the growth of cancer cells (Trerotola, Cantanelli et al. 2013). Therefore Trop-2 is a "non-oncogene", to the overexpression of which the tumor cell is addicted for growth (Trerotola, Cantanelli et al. 2013). This growth stimulation is independent from the histotype (carcinoma, sarcoma) and from the species (human, murine), indicating that the signalling network through which Trop-2 acts (Guerra, Trerotola et al. 2013) is highly conserved (Trerotola, Cantanelli et al. 2013) (S. Alberti "Anti-Trop-2 monoclonal antibodies and uses thereof in the treatment and diagnosis of tumors" - PCT/IT2009/000035).

This signalling network will now be further defined by the Authors, in order to identify effector molecules activated specifically and differentially in tumors that express Trop-2, which be used as targets for Trop-2-dependent anticancer therapy.

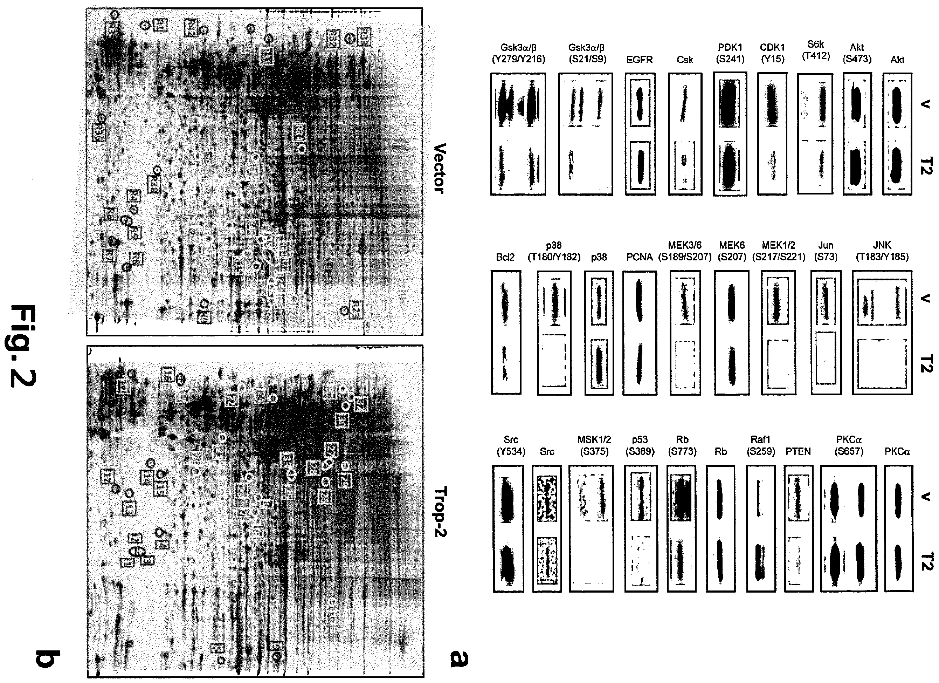

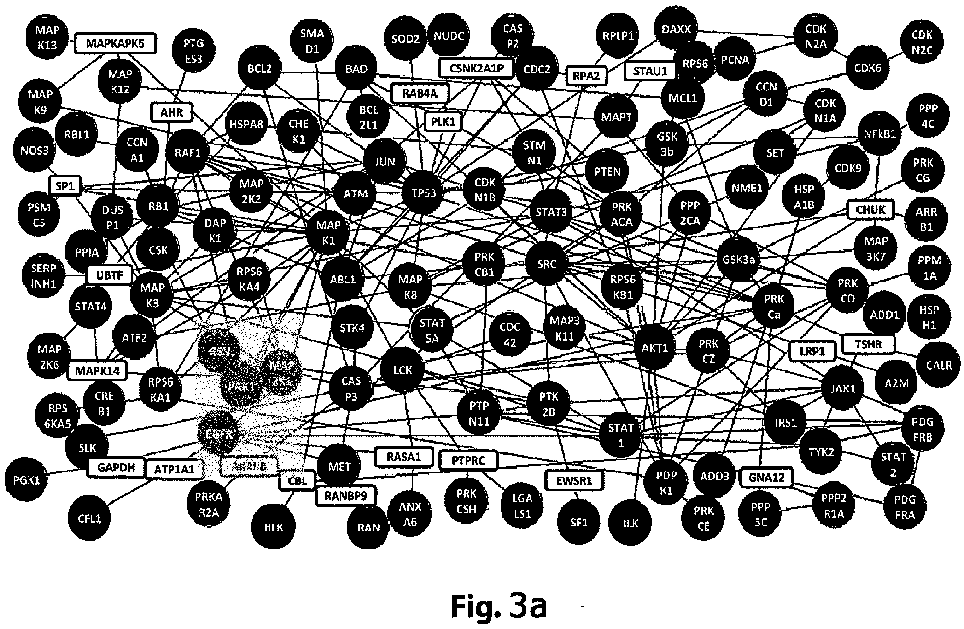

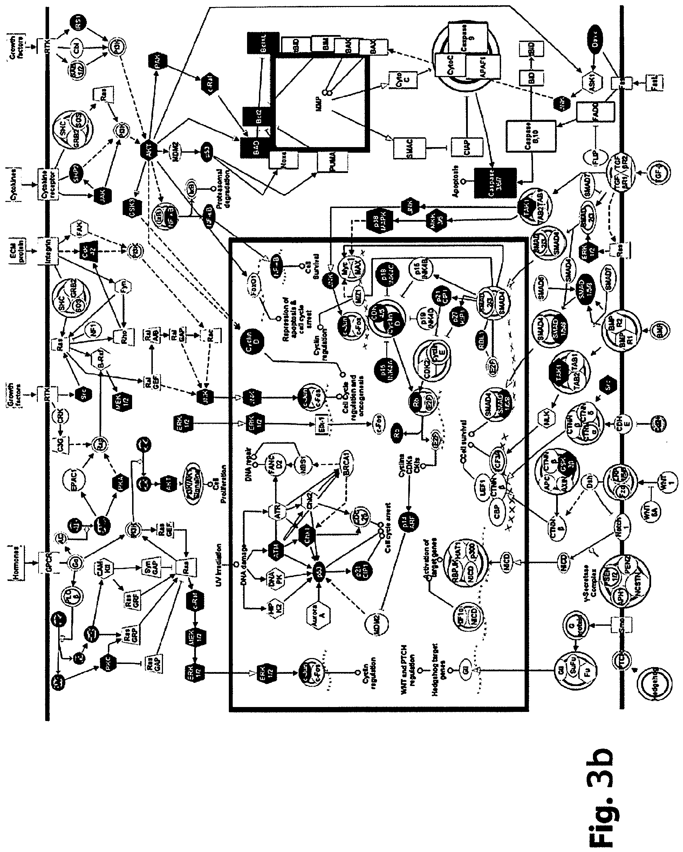

Electron microscopy analyses carried out by the Authors show that Trop-2 is localized in aggregates in macrovilli at the apical surface of the cell, in villi that extend between cells and in discrete regions of the membrane (Figure 2). Further investigations have identified these aggregates as tetraspanin networks, structural platforms in the membrane that can direct molecular networks of interaction between signalling proteins. The systematic analysis by confocal microscopy of the interactions between Trop-2 and specific markers for these macromolecular platforms (Barreiro, Zamai et al. 2008) revealed co-localization of Trop-2 with tetraspanins and molecules associated with them (CD9), both in intact cells (Figure 3a) and in "cell footprints" (Hakomori 2002)) (Figure 3c). It was also demonstrated that there is a direct physical interaction between Trop-2 and CD9, CD81, CD82, CD151 in human and murine cells, using techniques of co-capping and co-immunoprecipitation (Figure 3b, e). On the other hand, Trop-2 did not colocalize with caveolin, characteristic marker of lipid rafts (Figure 3a-c).

It is known that tetraspanin binding to their partners is cholesterol-dependent (Hakomori 2002): further confirmation of the fact that Trop-2 is an integral component of the tetraspanin platforms is provided by the ability of methyl-β-cyclodextrin and digitonin, which are respectively able to remove and precipitate cholesterol from cell membranes (Hakomori 2002, Charrin, 2003 #17269), to induce the release of Trop-2 in the culture medium and the precipitation of Trop-2 from lysates of cell membranes in BRIJ (Le Naour, Andre et al. 2006) (Figure 3f).

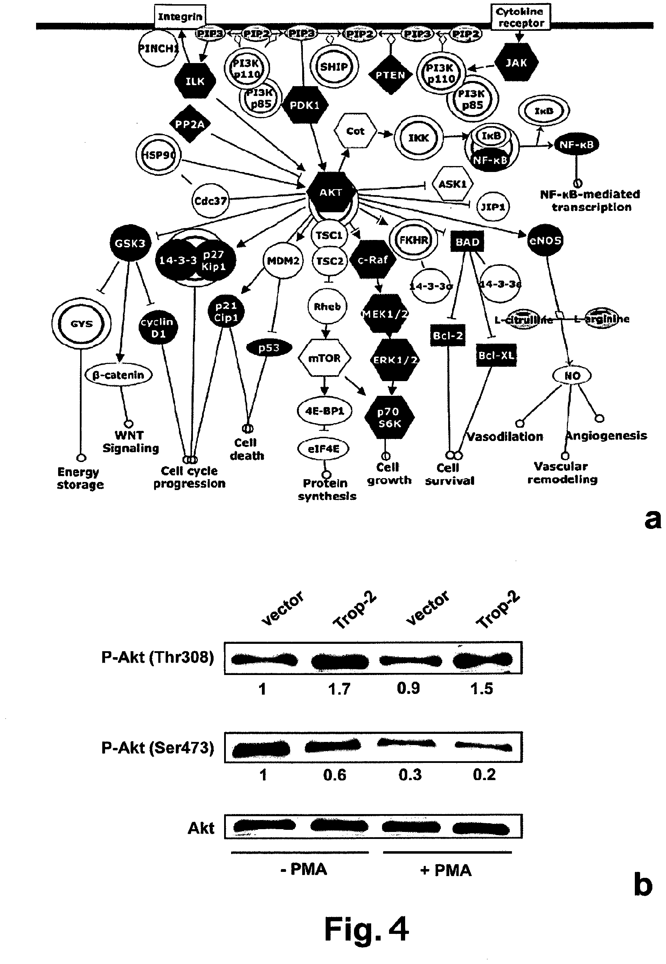

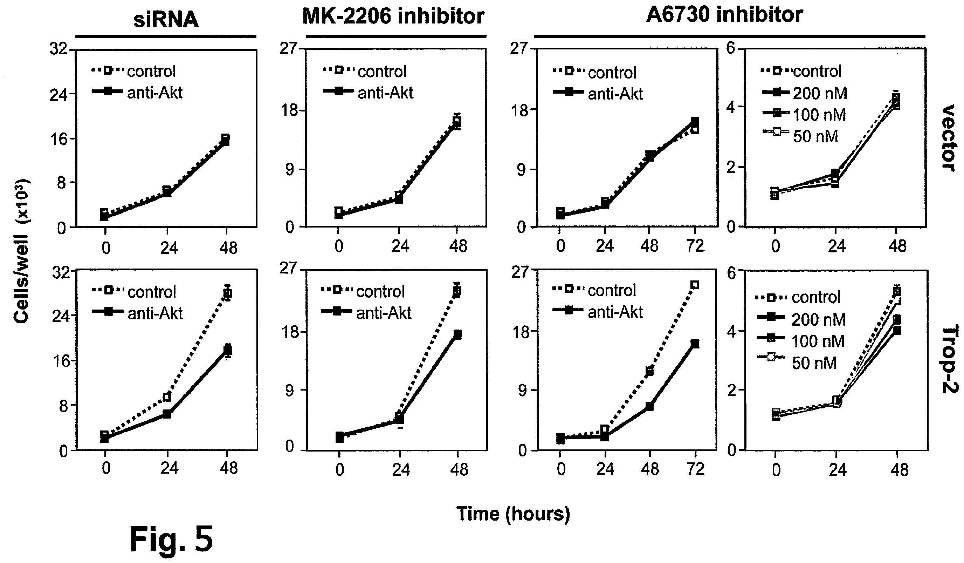

The tetraspanin networks provide support for diverse cell surface receptors which activate lipids, protein kinases and intracellular effectors (Hemler 2003; Le Naour, Andre et al. 2006). Accordingly, the Authors of the present invention had already shown that Trop-2 is able to modulate the expression of trans-membrane tyrosine-kinase receptors (PDGFR, Met, Ret, VEGFR), tyrosine and serine / threonine kinases, phosphatases , regulatory molecules of cell cycle and apoptosis (Guerra, Trerotola et al. 2013) (S. Alberti "Anti-Trop-2 monoclonal antibodies and uses thereof in the treatment and diagnosis of tumors" - PCT/IT2009/000035). These results are here extended with new proteomic analyses (Figure 4). The application of bioinformatic tools for the statistical analysis of protein-protein contacts (Minguez, Gotz et al. 2009) and for pathway analysis (Ingenuity Pathways Analysis software) allowed us to define the main signalling network of Trop-2 (Guerra, Trerotola et al. 2013) (Figure 5). The molecules driven by Trop-2 include signal transducers (transmembrane receptors, cytoplasmic tyrosine and serine / threonine kinases, phosphatases), components of the synthesis, folding and degradation of proteins, enzymes involved in the synthesis of nucleic acids and in the metabolism of carbohydrates). More than two thirds (114/165) of all the proteins modulated by Trop-2 (Figure 4) are connected by protein-protein contacts (Figure 5a). This network of protein-protein contacts has been identified as an important signalling pathway for cancer growth (Figure 5b), which includes the PKC, Akt/Gsk3β/S6K and ERK pathways, and the effectors NF-kB, Jun, CREB1 , STATs, Rb and p53 (Guerra, Trerotola et al. 2013).

These data have thus enabled the Authors of the present invention to demonstrate that PKCs are key mediators in the signalling network of Trop-2. Altogether, Trop-2 modulates 7 PKC substrates and 55 substrates / regulatory molecules of PKC (Figures 4 and 5). PKCα shows the highest increase in phosphorylation to its activatory site S657 in cells expressing Trop-2 (Figure 4a), indicating that its activation is Trop-2-dependent. This is consistent with the activation of the ERK signaling pathway and with the modulation of PP2A (phosphatase involved in inactivating PKCα) and of PDK1 (which phosphorylates PKCα in its activatory sites) induced by Trop-2. PKCα is frequently overexpressed in tumors, and guides the signalling processes involved in cancer. PKCα is also one of the main effectors of CD9 (Zhang, Bontrager et al. 2001), and is recruited to tetraspanin complexes (Zhang, Bontrager et al. 2001; Rosse, Linch et al. 2010), where it is activated by diacylglycerol (DAG) (Parekh, Ziegler et al. 2000).

On the basis on these indications, the Authors of the present invention have shown that CD9 microdomains function as connectors between PKCα and Trop-2: PKCα co-immunoprecipitates with CD81 and CD151 tetraspanins and to a greater extent with CD9 (Figure 3e). In a similar manner, Trop-2 co-immunoprecipitates with CD9, CD81, CD82 and CD151 (Figure 3e). Furthermore CD9, Trop-2 and PKCα colocalize in distinct membrane subdomains and this colocalization is phosphorylation-dependent (Figure 3c, d), thus demonstrating that both Trop-2 and PKCα are recruited to tetraspanin microdomains through interaction with CD9.

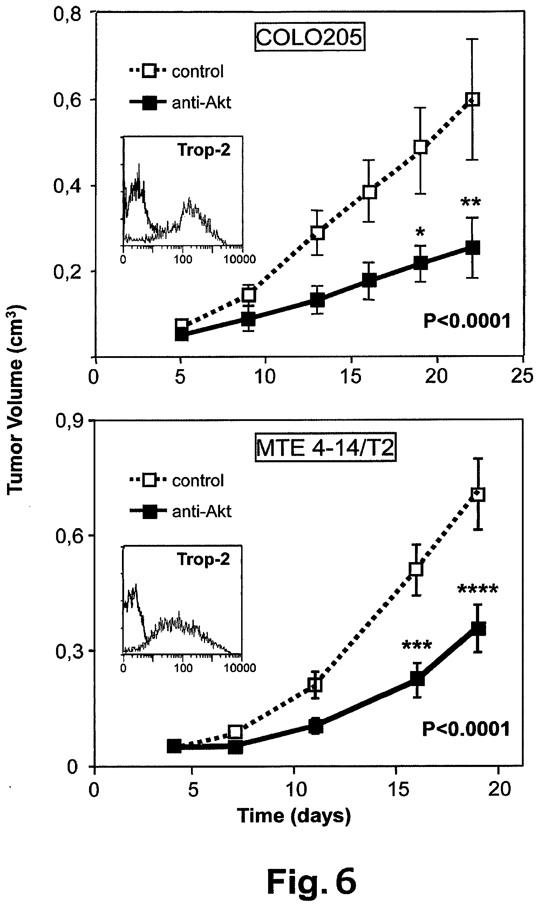

Bioinformatic analyses of this signalling network has then led the Authors to identify the Akt signalling pathway as key pathway in the stimulation of cell growth by Trop-2, through the GSK3 and S6K arms, but not that of mTOR (Fig. 6). Akt is the major downstream effector of PI3K. Akt T308 and S473 activation sites are targets for phosphorylation. The rictor-mTOR complex, together with ILK (Koul, Shen et al. 2005), phosphorylates Akt on S473 (Sarbassov, Guertin et al. 2005), but has no role in the T308 phosphorylation and in the activation of GSK3 and S6K effectors (Jacinto, Facchinetti et al. 2006). Consistent with this, Trop-2 induces the phosphorylation of Akt-T308, but not of Akt-S473 (Figure 6b), and modulates the phosphorylation of GSK3 and S6K (Figure 4a), but not of mTOR. The down regulation of ILK, previously demonstrated on proteomic chips by the Authors of the present invention, and the reduction, further increased by PMA, of Akt-S473 phosphorylation by Trop-2 (Figure 6b), is consistent with the down-regulation of ILK and of the Akt-S473 phosphorylation which is induced by PKC (Wen, Huang et al. 2003). On the other hand, Trop-2 induces the Akt-T308 phosphorylation (Figure 6b), which is consistent with the stoichiometric reduction, also previously demonstrated on proteomic chips, of PP2A catalytic and regulatory subunits (PPA is a Ca²⁺-dependent phosphatase that acts on T308) (Freeley, Park et al. 2007), rather than with the induction of PDK1 (Figure 4a) Consistent with a PKCα -independent mechanism, PMA has no effect on Akt-T308 phosphorylation levels (Figure 6b).

The interaction of Trop-2 with tetraspanin membrane complexes and in particular with CD9 activates a complex downstream signalling network that has the Akt signalling pathway as key component, and stimulates cell proliferation.

The Authors then studied the effect of selective inhibition of CD9 and Akt in proliferating cells expressing Trop-2. The downregulation of CD9, mediated by siRNA (Figure 7a, b) results in a striking reduction in the proliferation of MTE 4-14 cells expressing Trop-2, but has essentially no impact on Trop-2-negative cells (Figure 7d). This reduction is comparable to that caused by the silencing of Trop-2 (Figure 7d), and is consistent with the essential role of Trop-2 in the growth of cancer cells. Moreover, considering that the expression of CD9 is not influenced by Trop-2 (Figure 7c), these results also indicate that CD9 is necessary for the stimulation of tumor growth by Trop-2, but is otherwise inactive on baseline cell growth. Akt inhibition, either by silencing or with specific drugs, induces a marked decrease in cell proliferation in cells expressing Trop-2, in a dose-dependent fashion, while it has no effect on control cells (Figure 8). Consistent with this, Trop-2 expressing tumors subcutaneously injected in animal models show a striking reduction of growth following treatment with specific drugs that inhibit Akt activity (Figure 9). Therefore Akt has a central functional role in mediating the Trop-2-dependent growth stimulus, and pharmacological inhibition of this molecule has an anti-cancer therapeutic effect in preclinical models.

### DESCRIPTION OF THE INVENTION

The Authors of the present invention have identified a novel signalling network that stimulates tumor growth and is specifically activated by Trop-2 through interaction with CD9 and activation of the Akt signaling pathway. Trop-2 overexpression dictates sensitivity to Akt and CD9 inhibitors, which are able to selectively stop the growth of the cells that express Trop-2. These results pave the way to an original mode of personalized anticancer therapy.

Therefore it is a specific embodiment of the present invention a method to predict *in vitro* the outcome of an anticancer therapy with drugs inhibiting the activity of a component of the Trop-2 signaling network, wherein said component is Akt said method comprising or consisting of determining the expression levels of Trop-2 protein or of the corresponding mRNA in a biological sample, an effective outcome occurring when an increase of the expression levels of Trop-2 protein or of the corresponding mRNA as compared to the levels in the corresponding normal tissues is detected.

In one embodiment, positivity for the biological marker Trop-2 means an increase of Trop-2 mRNA or protein equal to or greater than 10% in the tumor as compared to the corresponding normal tissues of the same subject.

In another embodiment the present invention provides a method for *in vitro* screening of candidate drugs for the treatment or prevention of cancer, or the inhibition of cancer cell growth, wherein such drugs are directed against a component of the Trop-2 signaling network, wherein said component is Akt. This method comprises the following steps: the compound to be tested, in sterile saline solution, is administered to cells expressing Trop-2 and not expressing Trop-2; in parallel the sterile saline solution alone is administered to cells expressing Trop-2 and not expressing Trop-2. The non-expressing cells in contact with the compound allow to control the specificity of action of the compound itsels, while cells expressing and not expressing Trop-2 in contact with the sterile saline solution alone allow to control the activity of the compound itself. Subsequently the biological activity of the compound is measured, i.e. the proliferation of the cells expressing Trop-2 in comparison with the cells not expressing Trop-2 subjected to the treatment and with the cells expressing and not expressing Trop-2, not subjected to the treatment, i.e. in contact with the saline solution alone. Finally the compound is selected that is able to reduce cell proliferation by at least 10% in the cells expressing Trop-2 and subjected to the treatment, in comparison with the proliferation of cells not expressing Trop-2 and subjected to treatment and to cells not subjected to treatment.

The present invention provides also a method for the in vivo screening of candidate drugs for the treatment or prevention of cancer, or the inhibition of cancer cell growth, where the said drugs are directed against a component wherein said component is Akt. This method comprises the following steps: the compound to be tested, in sterile saline solution, is administered to tumors which are positive (i.e. overexpressing) or negative (i.e. not overexpressing) for the biological marker Trop-2, in preclinical models, excluding humans; in parallel the sterile saline solution alone is administered to tumors which overexpress or not Trop-2. The non-overexpressing tumors in contact with the compound allow to control the specificity of action of the compound itsels, while tumors overexpressing or not Trop-2 in contact with the sterile saline solution alone allow to control the activity of the compound itself. Subsequently the biological activity of the compound is measured, i.e. the growth of the tumors overexpressing Trop-2 in comparison with the tumors not overexpressing Trop-2 subjected to the treatment and with the tumors overexpressing or not Trop-2, not subjected to the treatment, i.e. in contact with the saline solution alone. Finally the compound is selected that is able to reduce tumor growth by at least 10% in the tumors overexpressing Trop-2 and subjected to the treatment, in comparison with tumor growth of treated and untreated controls.

In addition, the present invention concerns a compound inhibiting the activity of a component of the Trop-2 signaling network, wherein said component is Akt for use in the treatment of tumours expressing levels of Trop-2 protein or corresponding mRNA higher than normal tissue.

Some already known inhibitory molecules are: MK-2206, A6730, Perifosine, GSK690693, GSK2110183, GDC-0068, AT7867, ARQ092, AZD5363, A-674563, PHT-427, PF-04691502, SureCN1078972, 842148-40-7, AC1NX3D3, MLS002702033, SureCN10005574, SureCN1559590, SureCN570829, SH-5, SH-6, Honokiol, Miltefosine, Triciribine phosphate (Akt inhibitors), K00598a, DAPH 2, SureCN238877 (EGFR inhibitors), SureCN4269573 (inhibitor of EGFR, c-RAF, Src), dasatinib (Src inhibitor), SureCN1518805, 1,9-Pyrazoloanthrone, AS-601245, aminopyridine deriv. 2 (JNK inhibitors).

Optionally the above compound can be used in combination with other therapies, such as chemotherapy, alkylating agents (such as mechlorethamine chlorambucil, cyclophosphamide, ifosfamide, melphalan, nitrosoureas, busulfan, dacarbazine (DTIC), temozolomide, thiotepa and altretamine), antimetabolites (such as 5-fluorouracil (5-FU),6-mercaptopurine (6-MP), capecitabine, cladribine, clofarabine, cytarabine, floxuridine, fludarabine, gemcitabine, hydroxyurea, methotrexate, pemetrexed, pentostatin, thioguanine), anti-tumor antibiotics (such as anthracyclines, actinomycin-D, bleomycin, mitomycin-C), topoisomerase inhibitors (such as topotecan, irinotecan, etoposide, teniposide, mitoxantrone), mitotic inhibitors (such as paclitaxel, docetaxel, ixabepilone, vinblastine, vincristine, vinorelbine, estramustine), corticosteroids (such as prednisone, methylprednisolone, dexamethasone), differentiating agents (such as retinoids, tretinoin/ATRA, bexarotene and arsenic trioxide), hormone therapy, targeted kinase inhibitors, the proteosome inhibitor bortezomib.

The compound according to the present invention for use according to the invention can be chosen from the group consisting of oligonuclotides, engineered molecules corresponding to Akt which act as "dominant negative" molecules, monoclonal antibodies, pharmacological inhibitors, small-molecule chemical compounds or combination thereof that have AKT as target.

As mentioned above, the inhibition of the activity of AKT can be obtained, as a non-limiting example, by means of corresponding engineered molecules, which act as "dominant negatives", i.e. molecules similar to the normal ones but devoid of biological activity; these dominant negative molecules replace the normal ones and abolish their function in the cellular processes. The inhibition of biological activity can also be obtained with specific monoclonal antibodies that bind to catalytic and / or regulatory sites of the normal molecules and inhibit their activity. The inhibition of biological activity can also be obtained with pharmacological inhibitors. One of the main examples are MK-2206 and A6730, pharmacological inhibitors of Akt, for which Trop-2 expression constitutes indication for anti-cancer use according to the present invention.

The present invention concerns also oligonucleotides (RNA or DNA, double o single stranded) able to inhibit the growth of tumors in patients, these tumors being positive for the biological marker Trop-2, by inhibiting the expression of a component of the signalling pathway of Trop-2, wherein said component is Akt.

As an example, a method to inhibit AKT expression consists in siRNAs targeted against AKT, wherein the target sequence in the sense strand is contained in the mRNA of AKT (NM_009652.3), including but not limited to the following sequences:
sense 5' GCACCTTTAT TGGCTACAA 3' (SEQ ID NO:5),
antisense 5' TTGTAGCCAA TAAAGGTGC 3' (SEQ ID NO:6),
and the corresponding oligonucleotides for the transcription into hairpin RNAs:
forward 5' GATCCCCGCA CCTTTATTGG CTACAATTCA AGAGATTGTA GCCAATAAAG GTGCTTTTTC 3' (SEQ ID NO:7),
reverse 5' TCGAGAAAAA GCACCTTTAT TGGCTACAAT CTCTTGAATT GTAGCCAATA AAGGTGCGGG 3'(SEQ ID NO:8);
   or
sense 5' GGAAGGTGAT TCTGGTGAA 3' (SEQ ID NO:9),
antisense 5' TTCACCAGAA TCACCTTCC 3' (SEQ ID NO:10)
and the corresponding oligonucleotides for the transcription into hairpin RNAs:
forward 5' GATCCCCGGA AGGTGATTCT GGTGAATTCA AGAGATTCAC CAGAATCACC TTCCTTTTTC 3'(SEQ ID NO:11),
reverse 5' TCGAGAAAAA GGAAGGTGAT TCTGGTGAAT CTCTTGAATT CACCAGAATC ACCTTCCGGG 3'(SEQ ID NO:12).

According to the invention sequences SEQ ID NO:5 to SEQ ID NO:12 can be used as single stranded oligonucleotides or as double stranded oligonucleotides. Said sequences can be inserted in a cloning vector.

Varughese et al. (2011a,2011 b, 2011c) and Raji et al. (2011) showed that Trop-2 is overexpressed in uterine and ovarian carcinomas and that these carcinomas are highly sensitive to immunotherapy with the hRS7 humanised anti- Trop-2 monoclonal antibody. In these cases the growth of Trop-2 expressing tumors can be inhibited by monoclonal antibodies that directly target Trop-2.

The patent application WO2011/130654 concerns the ability by FOX03a to predict the outcome of AKT inhibition in cancer, such outcome being positive (i.e. tumor inhibition) when FOX03 has a cytoplasmic localization. FOX03a has a cytoplasmic localization when it is phosphorylated; FOX03a is a direct substrate of the phosphorylation activity of AKT; hence cytoplasmic FOX03a is a direct indication that AKT is enzimatically active in the cell.

### INDUSTRIAL APPLICATION AND WAYS OF IMPLEMENTATION

The present invention can be applied to the clinic for the cure of patients bearing tumors, where Trop-2 overexpression constitutes a biological marker that guides the theraputic choice toward compounds and /or treatments targeted against a component of the signalling network of Trop-2, wherein such component is Akt. Another application of the present invention is in the screening of new drugs and treatments for cancer therapy, using cells and tumors that overexpress Trop-2 and are sensitive in vitro and in vivo to substances and / or treatments directed against Akt.

*TROP2* mRNA increase in tumor cells compared to the corresponding control cells can be quantified in cells in culture and biopsies consisting of fresh or frozen cells and tissues. The mRNA can be extracted and purified by one skilled in the art by applying available techniques. Kits and instrumentation for extraction and purification can be acquired from commercial sources. The detection and quantification of *TROP2* mRNA by cDNA synthesis and PCR or real-time quantitative PCR, as described in the present invention, can be carried out according to techniques known in the art. Reagents and instrumentation for real-time quantitative RT-PCR are available on the market.

Trop-2 protein increase in tumor cells compared to the corresponding control cells can be quantified in cells in culture and biopsies consisting of fresh, frozen or formalin-fixed cells and tissues, by means of immunohistochemistry, ELISA assays, Western blotting or other equivalent techniques known in the art. Trop-2-reactive antibodies, kits and equipment for the execution of these tests and measurements are available on the market.

The immortalized murine cell line MTE 4-14 (Naquet, Lepesant et al. 1989) can be transfected according to techniques known in the art. MTE 4-14 cells transfected with an expression vector for Trop-2 and with the empty vector can be grown in vitro and used for the screening of compounds targeted against a component of the signalling network of Trop-2, wherein such component is Akt. Cell proliferation can be measured by direct counting or staining for cellular constituents or metabolic assays, according to techniques known in the art.

Preclinical models to be used for the in vivo screening of compounds directed against a component of the signalling network of Trop-2, wherein such component is Akt consist of immunocompromised mice which are subcutaneously injected with cells overexpressing or not overexpressing Trop-2 (xenografts), and similar models, according to methods known in the art.

The present invention will be now described by way of illustration and example, according, but not limited, to, some of its preferred embodiments, with particular reference to the detailed examples and figures of the attached drawings.

### DESCRIPTION OF THE DRAWINGS

Figure 1. Trop-2 cytoplasmic tail determinants responsible for growth stimulation. (a) Secondary structure prediction of the C-terminal portion of Trop-2 (aa 275-323); negatively -charged amino acids are underlined. The prediction softwares which were used are indicated on the left. h (highlighted in dark gray): α-helix, e (highlighted in black): β-strand; c (highlighted in light gray): connecting region (loop). Consensus secondary structure according to the prediction is indicated at the bottom, with residue-by-residue scores from a 1 to 10 scale. Prediction accuracy score is ≥80% (cubic.bioc.columbia.edu). (b) Sequence of the Trop-2 cytoplasmic tail mutants; the normal sequence (wt) is shown at the top. The HIKE domain is highlighted in the gray box; the position of the putative serine phosphorylation sites is highlighted in white. S303A, S322A: point mutations with change of serines 303 and 322, respectively, to alanine; E→K: point mutations with change of glutamic acids 310, 313, 316 and 320 in lysines; ΔHIKE: deletion of the HIKE region; Δcyto: deletion the entire cytoplasmic tail. (c) Effect of the Trop-2 cytoplasmic tail mutations on Trop-2 dependent growth. Growth curves of MTE 4-14 cells transfected with the empty vector or with wt Trop-2 or mutant Trop-2 as indicated. Difference between wt and mutant Trop-2: P <0.0001 (two-way ANOVA). Stars indicate statistically significant differences for individual time points (Bonferroni test for multiple comparisons): **** ≤ 0.0001. Bars: standard error of the mean.
Figure 2: Trop-2 signalling clusters. Electron microscopy analysis of Trop-2 expression. (a) MCF7 breast cancer cells stained with anti Trop-2 antibodies conjugated to gold nanospheres (black dots). Trop-2 is identified in elongated villar projection and macrovilli (arrowheads) and in discrete membrane domains. White circles (30 nm in diameter) are centered on individual gold particles and encompass the areas with the highest probability to find antibody molecules bound to Trop-2. Bar: 50 nm. (b) Trop-2-expressing cells stained with anti-Trop-2 antibodies conjugated to immuno-peroxidase (dark granular deposits). Trop-2-transfected 293 cells (i), OVCA-432 ovarian cancer cells (ii), MCF-7 breast cancer cells (iii). Trop-2 is localized in discrete membrane domains (arrowheads), at a cell-cell contact (i inset: magnified contact area), in villar projections (ii) and in discrete membrane domains (iii). The presence of Trop-2 is also detected at the level of intracytoplasmic vesicles (arrows).
Figure 3. Interaction of Trop-2 and PKCα with tetraspanin molecules and CD9. (a-d) Confocal microscopy analysis of intact cell membranes (a), membrane areas showing co-capping (b), footprints of cell membranes (c, d) of MTE 4-14 cells transfected with Trop-2 (a-c, d top) or with the empty vector (d bottom) stained simultaneously with anti-Trop-2 and anti-CD9 (member of tetraspanin webs) or anti-caveolin (negative control) (a-c) or anti-CD9 and anti PKCα (d) conjugated with fluorescent molecules. Areas of colocalization are indicated by arrowheads. (e) Co-immunoprecipitation (IP) of Trop-2, PKCα, CD9 and tetraspanins. Immunoprecipitation was performed using primary antibodies against murine CD9, CD81 and CD151 (MTE 4-14cells) or human CD9, CD82 and CD151 (MCF7 cells); the presence of Trop-2 and PKCα in immunoprecipites was detected by Western blot. IP with anti-Trop-2: positive control. (f) Trop-2/cholesterol association. Release of Trop-2 in the culture medium after treatment with methyl-β-cyclodextrin (MβCD) (left). Precipitation of Trop-2 with digitonin from BRIJ lysate (right). The treatments were performed on MTE4-14 cells transfected with Trop-2, and the presence of Trop-2 in the soluble and insoluble fractions was assessed by Western blot.
Figure 4. The Trop-2 proteome. (a) Amount and phosphorylation of signaling molecules in MTE 4-14 transfectants, measured by Western blot. V: vector alone; T2: Trop-2. The panels with four bands show two pairs of independent measurements. (b) 2D gels of protein extracts from MTE 4-14 cells transfected with the vector alone (left) or Trop-2 (right); circles indicate proteins repressed (R, left) or induced (I, right) by Trop-2 expression.
Figure 5. Trop-2 signalling network. (a) Network of protein-protein interactions driven by Trop-2 (SNOW analysis). Circles: all the proteins modulated by Trop-2 that can physically interact with each other (114 of the 165 proteins identified). Rectangles: "connectors" between two proteins. Lines ("edges") represent protein-protein contacts. (b) Cancer signaling network drived by Trop-2. The proteins modulated by Trop-2 (in black) were mapped using the program Ingenuity Pathway Analysis. Hexagons: kinases; trapezoids: small G proteins and their interactors; rectangles: apoptotic factors; ovals: transcription factors/ chromatin remodelling proteins; double circles: protein complexes. Highlighted in gray are signalling molecules which are not proteins. Thick gray lines: cell membrane; gray boxes: nuclear and mitochondrial compartments. P = 6.19x10-45.
Figure 6. Control of Akt signalling network by Trop-2. (a) Map of the Trop-2/Akt signalling network. The proteins which are modulated by Trop-2 (in black) were mapped using the Ingenuity Pathway Analysis software. Hexagons: kinases; rectangles: apoptotic factors; diamonds: phosphatases; ovals: transcription factors/ chromatin remodelling proteins; double circles: protein complexes. Highlighted in gray are signalling molecules which are not proteins. Thick gray line: the cell membrane. (b) Modulation of the phosphorylation levels at Akt threonine 308 and serine 473 by Trop-2. Western blot analysis of MTE 4-14 cells transfected with the vector alone or with Trop-2, in the presence or absence of PMA; for each band is indicated the intensity, normalized with respect to the control.
Figure 7. Trop-2 dictates sensitivity to CD9 inhibitors. (a, b) Control of the efficiency of CD9 silencing in MTE4-14/vector and MTE4-14/Trop-2. (a) Measurement by quantitative real-time RT-PCR of the mRNA levels of CD9 24 hours after transfection with an ineffective siRNA (control) or the specific anti-CD9 siRNA. Bars: standard deviation of the measurements. (b) Flow cytometry analysis of CD9 levels 48 hours after transfection with an ineffective siRNA (control) or the specific anti-CD9 siRNA. White profiles: control antibody, unstained; gray profiles: anti-CD9 antibody conjugated to Alexa-488. (c) Flow cytometry analysis of CD9 levels in murine or human cells cells stably transfected with Trop-2 (MTE 4-14/Trop-2, IGROV/Trop-2, KM12-SM/Trop-2) or endogenously expressing Trop-2 (HCT116U5.5, MCF7, HT29). White profiles: control antibody, unstained; gray profiles: anti-CD9 antibody conjugated to Alexa-488. (d) Growth curves of MTE4-14/vector (left) and MTE 4-14/Trop-2 (right) transfected with an ineffective siRNA (control) or the specific anti-CD9 or anti-Trop-2 siRNA. Bars: standard error of the mean.
Figure 8. Trop-2 dictates sensitivity to Akt inhibitors. Growth curves of MTE 4-14 cells transfected with the vector alone or with Trop-2 and treated with ineffective siRNA (control) or specific anti-AKT siRNA (left), or with solvent alone or pharmacological inhibitors of Akt (center, right), using a dose of 400 nM or different doses as indicated. Bars: standard error of the mean.
Figure 9. Inhibition of growth of Trop-2 expressing tumors, through inhibition of Akt. In vivo growth curves of Colo205 colon cancer cells (top) or MTE 4-14 cells transfected with Trop-2 (bottom) treated with the solvent alone or with the MK-2206 pharmacological inhibitor of Akt. MTE 4-14 control cells transfected with the empty vector does not have tumorigenic ability in this model. Difference between control and treated tumors: P <0.0001 (two-way ANOVA). Stars indicate statistically significant differences for individual time points (Bonferroni adjustment for multiple comparisons): * ≤ 0.05, ** ≤ 0.01, *** ≤ 0.001 ≤ 0.001 ****. Bars: standard error of the mean. Insets, flow cytometry analysis of Trop-2 expression in tumor cells and transfectants: white profiles, control antibody, unstained; gray profiles, anti-Trop-2 conjugated to Alexa-488

### EXAMPLES

### Cell cultures

The human MCF-7 and OVCA-432 cell lines were grown in RPMI 1640 medium (GibcoBRL, Paisley, Scotland). The human 293 and KM12SC and the murine immortalised MTE 4-14 (Naquet, Lepesant et al. 1989) and L cell lines were maintained in DMEM. All media were supplemented with 10% fetal bovine serum (GibcoBRL), with 100 IU/ml penicillin and 100 µg/ml streptomycin (Euroclone, Milano, Italy).

### DNA transfection

Cells were transfected with DNA in Lipofectamine 2000 or LTX (Invitrogen, Carlsbad, CA) following the manufacturer instructions. Stable transfectants were selected in G-418-containing medium.

### Immunofluorescence analysis.

Cells plated on glass coverslips were stained with the indicated antibodies conjugated with Alexa Fluor-488/546/633, or with the same unconjugated antibodies detected by a secondary antibody conjugated with Alexa Fluor-488/546/633. Cells were either staained alive, before fixation, in medium with 10% FBS, at 37 ° C for 5 minutes, or stained after fixation, by incubation with the relevant antibodies in PBS at room temperature for 30 minutes. Cells were fixed with 4% paraformaldehyde in PBS for 20 min. Permeabilisation and blocking of non-specific interactions were performed with 10% FCS, 0.1% saponin. The slides were then washed in PBS and mounted to be analyzed by LSM-510 META confocal microscope (Zeiss, Oberkochen, Germany).

### Cell footprints

"Cell footprints" are membrane remnants on tissue culture dishes after detachment of cells with EDTA (Hakomori 2002). Cell treatment and immunofluorescence analyses were conducted essentially as described (Hakomori 2002).

### Co-immunoprecipitation assays

Cells were lysed in BRIJ buffer (150 mM NaCl, 50 mM Tris-HCl, pH 7.5, 1 mM MgCl2, 1 mM CaCl2, 1% BRIJ, protease inhibitors). Cell lysates were cleared by incubation with Protein-G Sepharose (GE Healthcare, Piscataway, NJ) at 4 °C for 1 h. Cleared lysates were incubated with primary antibodies against putative Trop-2 interactors at 4 °C for 2 h, followed by incubation with Protein G Sepharose (at 4 °C, 1 h). Protein complexes were eluted from the resin with 0.1 M glycine, pH 2.5, and probed by Western blotting with specific antibodies as indicated.

### Depletion and precipitation of cholesterol from cell membranes.

To deplete cell membranes of cholesterol, intact cells were washed three times in PBS to remove serum and incubated in DMEM with 10 mM methyl-β-cyclodextrin at 37 °C for 45 min. Control cells were processed in parallel in the absence of methyl-β-cyclodextrin. Samples were centrifuged at 2000 g to remove cells and cell debris. Proteins in the supernatant were precipitated in trichloroacetic acid and analysed by Western blotting.

Cells were also harvested by scraping and lysed in buffer with 1% BRIJ. The supernatant was mixed with either 1/10 (vol/vol) methanol (control) or 1/10 (vol/vol) 10% digitonin in methanol, and incubated on ice for 10 min. Cell membranes were recovered by centrifugation at 12,000 g for 15 min. The pellet was washed in cold acetone, resuspended in Laemmli buffer and analysed by Western blotting.

### Immuno-electron microscopy.

For immuno-gold electron microscopy (EM), cells were fixed in 4% formaldehyde, 0.05% glutaraldehyde, 0.15 M HEPES, pH 7.3, for 10 min at 37 °C, and post-fixed for 50 min in 4% paraformaldehyde, 0.15 M HEPES, pH 7.3 at room temperature, as described (Polishchuk, Polishchuk et al. 2000). Cationised gold, protein A-gold, and gold-conjugated anti-rabbit antibodies (10 nm colloidal gold particles) were from British BioCell (Cardiff, UK). Immunoperoxidase EM was performed as described (Brown and Farquhar 1989). Estimates of minimal labelled areas were performed assuming an average length of antibody molecules of 11 nm.

### In vitro cell-growth assays.

MTE 4-14 cells transfected with the empty vector or with Trop-2 were seeded at 1.5-3×10³ cells/well in 96-well plates (five replica wells per data point). Cell numbers were quantified by the MTT colorimetric assay (Roche Molecular Biochemicals, Mannheim, Germany) or by staining with crystal violet. Cell numbers were normalised against a reference standard curve of serially diluted cell samples.

### Antibodies.

Rabbit polyclonal anti-Trop-2 antisera were generated by subcutaneous immunisation with recombinant Trop-2, that had been produced in bacteria (Fornaro, Dell'Arciprete et al. 1995). Trop-2-reactive antibodies were purified by binding to recombinant Trop-2 immobilized onto NHS-Sepharose columns (Pharmaci, Uppsala, Sweden), and eluted with 0.2 M glycine, pH 2.5. The goat anti-Trop-2 polyclonal antibody AF650 was obtained from R&D Systems, Inc. (Minneapolis, MN). Additional antibodies directed against murine molecules were: rat monoclonal anti-CD9 (sc-18869); rabbit monoclonal anti-Akt (ser473) (D9E) (Cell Signal Technology, MA); goat polyclonal anti-CD151 (sc-18753), anti-phospho-PKCa (S657) (sc-12356), anti-Akt (sc-1618); rabbit polyclonal anti-caveolin-1 (sc-894), anti-PKCa (sc-208), anti-phospho-Akt (Thr 308) (sc-16646-R) (Santa Cruz Biotechnology, Santa Cruz, CA); hamster polyclonal anti-CD81 (GTX75430) (GeneText, Irvine, CA). Mouse monoclonal antibodies directed against corresponding human molecules were: anti-CD9 (14-0098), anti-CD98 (14-0982) (eBioscience, San Duego, CA), anti-CD151 (H00000977-M02) (Abnova, Taiwan, China) and anti-CD81 (TS81) (generously supplied by Dr. F. Lanza). Secondary Alexa Fluor-488/546/633 conjugated antibodies were from Invitrogen.

### Pharmacological inhibitors.

Cells were treated with 50-100-200-400 nM A6730_SIGMA (Sigma Technical Company, St Louis, MO 63178-9916) or with 400nM MK-2206 (Selleck Chemicals, Houston, TX) to inhibit Akt. Stock solutions were prepared in DMSO and diluited in ethanol or water, with the final concentration of DMSO and ethanol never exceeding 0.1% and 1% respectively. Control cells received vehicle alone. A second treatment was performed 24 hours after seeding.

### In vivo models: xenografts in athymic nude mice

MTE 4-14 immortalized cell line transfected with Trop-2 (Alberti, Nutini et al. 1994), or Colo205 colon cancer cells, endogenously expressing Trop-2, were injected subcutaneously (4x10⁶ cells/injection) into nude mice groups (8 weeks females, CD1-Foxn1 nu/nu mice (Charles River Laboratories, Calco, Lecco, Italy). MTE 4-14 cells transfected with Trop-2 were co-injected with Matrigel 1:3 vol/vol (growth factor reduced matrix, BD Biosciences, Franklin Lakes, NJ USA). A stock solution of MK-2206 in DMSO was diluted at the time of use in apyrogenic saline solution, to a final concentration of 0.7 mg/ml, and the drug was administered intraperitoneally at a dose of 0.35 mg/mouse every day, starting from the time in which the tumors became palpable. Control group mice were treated with the vehicle alone. Mice were visually inspected every 5-7 days and the major and minor diameters of the experimental tumors were measured. The tumor volume was calculated using the formula for the volume of ellipsoid (Dxd2/2). MTE 4-14 cells transfected with the empty vector were not tumorigenic in this model. Procedures involving animals and their care were conducted in compliance with institutional guidelines, national laws and international protocols (D.L. No.116, G.U., Suppl. 40, Feb.18, 1992; No. 8, G.U., July, 1994; UKCCCR Guidelines for the Welfare of Animals in Experimental Neoplasia; EEC Council Directive 86/609, OJ L 358. 1, Dec.12, 1987; Guide for the Care and Use of Laboratory Animals, United States National Research Council, 1996).

### Antibody-mediated co-capping.

Cells were detached from culture plates using trypsin, and incubated with the primary anti- Trop-2 antibody (T16) (Alberti, Miotti et al. 1992) for 20 min on ice. After washing, cells were incubated with the secondary Alexa Fluor 488-conjugated antibody for 10 min at 37 °C, to cross-link target molecules and to induce capping of the antigen-antibody complex (Levy and Shoham 2005). The 'capped' cells were fixed with 1% paraformaldehyde for 10 min at room temperature. The paraformaldehyde was quenched with FBS, and cells were stained with antibodies against the membrane proteins of interest.

### Western blot analysis.

Western blotting was performed as previously described (El Sewedy, Fornaro et al. 1998). In brief, cell lysates were analyzed by electrophoresis on denaturing polyacrylamide gel (SDS-PAGE) and transfered onto nitrocellulose filters. Filters were incubated with the relevant primary antibodies (Abcam, Cambridge, UK; Santa Cruz) in TBS with 5% non-fat dried milk. Hybridized filters were washed in TBS, 0.1% Tween-20. Antibody binding was revealed by chemiluminescence (ECL, Amersham, Aylesbury, UK), using horseradish-peroxidase-conjugated anti-mouse or anti-rabbit secondary antibodies (Calbiochem, La Jolla, CA). Signal intensity was quantified with NIH-image 1.62, using as reference a Kodak gray-scale standards power curve (www.kodak.com).

### Plasmids

The pEYFP expression vector was obtained from Clontech (Palo Alto, CA). A corresponding vector devoid of the coding sequence of EYFP was used to express wild-type or mutagenized Trop-2 cDNAs. The coding sequence of the wild-type human *TROP2* was amplified by PCR with the forward primer 5' GCGATTCTCG AGTCCGGTCC GCGTTCC 3' (SEQ ID NO:13) and with the reverse primer 5' GCGCCGGTAC CAAGCTCGGT TCCTTTC 3'(SEQ ID NO:14) and subcloned into the pEYFP-N1 vector. The expression vectors pCMV-SPORT6 and CD316 pCMV-SPORT6 were obtained from Imagenes (Berlin, Germany). The chimeric proteins between CD9 (EcoRI/BamHI), CD316 (EcoRI/Agel) and mCherry were constructed following standard procedures.

### siRNA.

Four design strategies were used, following the procedures according to: Tuschl criteria (Elbashir, Harborth et al. 2001): Invitrogen (rnaidesigner.invitrogen.com/rnaiexpress/), Whitehead Institute web site (jura.wi.mit.edu/bioc/siRNAext/) (Semizarov, Frost et al. 2003); Sonnhammer (sonnhammer.cgb.ki.se/siSearch/) (Chalk, Wahlestedt et al. 2004). The siRNAs that were chosen were the ones identified by more than one of the above methods, or considered optimal by at least one of them. The coding sequences for hairpin RNAs (shRNAs) obtained from the corresponding siRNAs (Elbashir, Harborth et al. 2001), specific for the genes of interest, have been subcloned in the pSUPER vector (Brummelkamp, Bernards et al. 2002), under the control of the promoter for the RNA polymerase-III H1 gene.

The inhibition of Akt has been obtained by means of anti-AKT siRNAs with target sense sequence 5' GCACCTTTAT TGGCTACAA 3' (SEQ ID NO:5) and antisense sequence 5' TTGTAGCCAA TAAAGGTGC 3' (SEQ ID NO:6), as an example included in the following sequences for the transcription of hairpin RNA:
forward 5' GATCCCCGCA CCTTTATTGG CTACAATTCA AGAGATTGTA GCCAATAAAG GTGCTTTTTC 3' (SEQ ID NO:7),
reverse 5' TCGAGAAAAA GCACCTTTAT TGGCTACAAT CTCTTGAATT GTAGCCAATA AAGGTGCGGG 3'(SEQ ID NO:8);
or target sense sequence 5' GGAAGGTGAT TCTGGTGAA 3' (SEQ ID NO:9), antisense sequence 5' TTCACCAGAA TCACCTTCC 3' (SEQ ID NO:10), as an example included in the following sequences for the transcription of hairpin RNA:
forward 5' GATCCCCGGA AGGTGATTCT GGTGAATTCA AGAGATTCAC CAGAATCACC TTCCTTTTTC 3'(SEQ ID NO:11),
reverse 5' TCGAGAAAAA GGAAGGTGAT TCTGGTGAAT CTCTTGAATT CACCAGAATC ACCTTCCGGG 3'(SEQ ID NO:12).

The corresponding constructs were transiently transfected into MTE 4-14 cells, and their effect on cell proliferation was evaluated. The levels of transcripts after silencing were quantified by real-time RT-PCR. siRNAs directed against irrelevant targets were used as negative controls, chosen after thorough verification of the absence of spurious effects on cell growth. The corresponding sequences are given below; for each siRNA the corresponding gene is indicated, with the identification code according to the RefSeq database (GenBank).
siRna directed against murine Co-029/Tspan8 (NM_146010.2), used as negative control for human cells, having the target sequence sense 5'CTTTCAAACC TGAGTATAA 3'(SEQ ID NO:15), antisense 5' TTATACTCAG GTTTGAAAG 3' (SEQ ID NO:16), as an example included in the following sequences for the transcription of hairpin RNA:
forward 5' GATCCCCCTT TCAAACCTGA GTATAATTCA AGAGATTATA CTCAGGTTTG AAAGTTTTTG GAAA 3' (SEQ ID NO:17),
reverse 5' AGCTTTTCCA AAAACTTTCA AACCTGAGTA TAATCTCTTG AATTATACTC AGGTTTGAAA GGGG 3'(SEQ ID NO:18).
siRNA directed against human CD133 (NM_006017.2), used as negative control for murine cells, having the target sequence sense 5' CTTGACAACG TTAATAACG 3' (SEQ ID NO:19), antisense 5' CGTTATTAAC GTTGTCAAG 3' (SEQ ID NO:20), as an example included in the following sequences for the transcription of hairpin RNA:
forward 5' GATCCCCCTT GACAACGTTA ATAACGTTCA AGAGACGTTA TTAACGTTGT CAAGTTTTTG GAAA 3' (SEQ ID NO:21),
reverse 5' AGCTTTTCCA AAAACTTGAC AACGTTAATA ACGTCTCTTG AACGTTATTA ACGTTGTCAA GGGG 3' (SEQ ID NO:22).
siRNA directed against human CD316 (NM_052868.2), used as negative control for murine cells, having the target sequence sense 5' TGCAGGAAGTG GTGGGAAT 3' (SEQ ID NO:23), antisense 5' ATTCCCACCA CTTCCTGCA 3' (SEQ ID NO:24), as an example included in the following sequences for the transcription of hairpin RNA:
forward: 5' GATCCCCTGC AGGAAGTGGT GGGAATTTCA AGAGAATTCC CACCACTTCC TGCATTTTTG GAAA 3' (SEQ ID NO:25),
reverse 5' AGCTTTTCCA AAAATGCAGG AAGTGGTGGG AATTCTCTTG AAATTCCCAC CACTTCCTGC AGGG 3' (SEQ ID NO:26).
*TROP2* silencing was obtained as previously described (Trerotola, Cantanelli et al. 2013).

### Mutagenesis of the cytoplasmic domain of Trop-2

The mutants of the cytoplasmic domain of Trop-2 were obtained by PCR with the following primers:
Trop-2 Δcyto
forward 5' CTCGGATCCA CCATGGCTCG GGGCCCC 3' (SEQ ID NO:27),
reverse 5' CTCGAATTCC CGGTTGGTGA TCACCAG 3' (SEQ ID NO:28).
Trop-2 E→K
forward 5' GCGGAATTCC GTCCGGTCCG CGTTCCT 3' (SEQ ID NO:29),
reverse 5' GCGTCTAGAC TACAAGCTCG GTTTCTTTCT CAACTTCCCC AGTTTCTTGA TCTTCACCTT CTTG 3' (SEQ ID NO:30).
Trop-2 ΔHIKE
forward 5' GCGGAATTCC GTCCGGTCCG CGTTCCT 3' (SEQ ID NO:31),
reverse 5' GCGTCTAGAC TACAAGCTCG GTTCCTTTCT CAACTCCCCC AGTTCCTTGA TCTCCACTCT CCGGTTGGTG ATCAC 3' (SEQ ID NO:32).
Trop-2 S303A
forward 5' CTCGGATCCA CCATGGCTCG GGGCCCC 3' (SEQ ID NO:33),
reverse 5' GCGTCTAGAC TACAAGCTCG GTTCCTTTCT CAACTCCCCC AGTTCCTTGA TCTCCACCTT CTTGTACTTC CCCGCCTTTC TCCGG 3' (SEQ ID NO:34).
Trop-2 S322A
forward 5' CTCGGATCCA CCATGGCTCG GGGCCCC 3' (SEQ ID NO:35),
reverse 5' GCGTCTAGAC TACAAGGCCG GTTCCTTTCT CAACTCCCC 3' (SEQ ID NO:36).
All amplified regions were completely sequenced to verify the absence of mutations induced by Taq-polymerase.

### Real-time quantitative RT-PCR.

Total RNA from the indicated cell lines was extracted in Trizol (Invitrogen) following the manufacturer's instructions. One µg of total RNA was used for each reverse transcription (RT) reaction, using the ImProm-II Reverse Transcriptase (Promega) according to standard protocols. Real-time quantitative PCR reactions were performed using an ABI-PRISM 7900HT Sequence Detection System (PE Applied Biosystems, Foster City, CA) with specific primers (Hs99999905; CD9: Mm00514275_g1; B2M: Mm00437762_m1) (Applied Biosystems) and the corresponding TaqMan® (Roche) fluorescent probes, according to the manufacturer instructions (Giulietti, Overbergh et al. 2001). Each sample was assayed in triplicate and the 2^{-ΔΔCT} method was used to calculate relative changes in gene expression (Livak and Schmittgen 2001). A more accurate base of 1.834 was used (Guerra, Trerotola et al. 2008), as 1.1 cycles are required to double the amplified material. The GAPDH and B2M housekeeping genes were used as an internal control. For each cDNA the ΔCT (CT, target gene-CT, GAPDH/B2M) was calculated: using least-squares linear regression analysis. As amplification efficiency was linear over the range of RNA amounts used, amplification curves were used to calculate cross-over point values for siRNA-treated samples. Each sample was routinely assessed for genomic DNA contamination by using non-retrotranscribed RNA as a templates for PCR reactions.

### 2D gel and mass-spectrometry.

A vision over the entire genome was obtained by 2D-PAGE performed as described (Swiss Institute of Bioinformatics (SIB) - http://us.expasy.org/ch2d/protocols/). The mass spectrometry analyses were performed at the University of York (www.york.ac.uk/depts/biol/tf/proteomics/).

### Pathway analysis and identification of Trop-2 signaling network

The proteome of Trop-2 was analyzed using the SNOW (snow.bioinfo.cipf.es / cgi-bin / snow.cgi) and Ingenuity Pathways Analysis (IPA, Ingenuity Systems, www.ingenuity.com) bioinformatics tools. SNOW (snow.bioinfo.cipf.es / cgi-bin / snow.cgi) builds networks of protein-protein interaction, mapping the protein over a reference interactome.The reference interactome is a set of pair-wise direct protein interactions, where the nodes are the proteins themselves and the connecting edges are the interaction events. A Trop-2-dependent interactome was built using the HPRD (Human Protein Reference Database), IntAct, BIND (Biomolecular Interaction Network Database), DIP (Database of Interacting Proteins) and MINT (Molecular Interaction Database) databases. These were used to build a *minimal connected network* (MCN). Topological networks were evaluated on the basis of the *node connections* degree (i.e. number of edges of each node); the *betweenness* (i.e. a measure of centrality of nodes and of their distribution); the *clustering coefficient* distribution (i.e. the connectivity of the neighbourhood of each node); the *number of components* of the network (i.e. the different groups of nodes that are generated in a network analysis); the *bicomponents* (i.e. the number of node groups connected to another node group by a single edge) and the *articulation points* (i.e. the edges that join bicomponents in a network). MCNs were identified using the Dijkstra's algorithm applying a stringent option that permits the introduction of only one external connector protein between any pair of input proteins analyzed. In this way it was possible to obtain a single network that combines 114 of the 165 proteins modulated by Trop-2 which were subjected to the analysis, with 22 connectors. Statistical significance was verified *versus* networks generated over the entire reference interactome and *versus* networks with random composition. For the analysis using the Ingenuity Pathways Analysis software, protein expression values, expressed as ratios between the normalized mean signal intensities between Trop-2 and control transfectants, were converted to fold-change values, where the negative inverse (-1/x) was taken for values between 0 and 1. As the data-set was filtered, no threshold cut-offs were imposed. All the molecules (Swiss-Prot identifiers) were mapped onto the Ingenuity Knowledge Base as focus points. The Ingenuity Knowledge Base organises protein interactions into an ontology format, as extracted by manual curation from published experimental results. Networks of focus molecules were generated by maximising specific connectivities, i.e. actual interconnectedness between molecules *versus* that with all molecules in the Knowledge Base. Networks were ranked by scores (negative log of the p-value calculated by right-tailed Fisher's exact test) that takes into account the number of eligible molecules in the network and its final size as well as the input data-set size and the total number of molecules in the Ingenuity Knowledge Base included in networks. The higher the score, the lower the chance of stochastic interaction networks. Focus molecules were then mapped onto the Ingenuity Canonical Pathways.

### Statistical analysis

The Two-way ANOVA test was used to compare the tumor growth curves (Rossi, Di Lena et al. 2008).

### References

Alberti, S. (1999). "HIKE, a candidate protein binding site for PH domains, is a major regulatory region of Gbeta proteins." Proteins 35: 360-363.
Alberti, S., S. Miotti, et al. (1992). "Biochemical characterization of Trop-2, a cell surface molecule expressed by human carcinomas: formal proof that the monoclonal antibodies T16 and MOv-16 recognize Trop-2." Hybridoma 11: 539-5.
Alberti, S., M. Nutini, et al. (1994). "DNA methylation prevents the amplification of TROP1, a tumor associated cell surface antigen gene." Proc. Natl. Acad. Sci. USA 91: 5833-7.
Balzar, M., I. H. Briaire-de Bruijn, et al. (2001). "Epidermal growth factor-like repeats mediate lateral and reciprocal interactions of Ep-CAM molecules in homophilic adhesions." Mol. Cell. Biol. 21(7): 2570-80.
Barreiro, O., M. Zamai, et al. (2008). "Endothelial adhesion receptors are recruited to adherent leukocytes by inclusion in preformed tetraspanin nanoplatforms." J Cell Biol 183(3): 527-42.
Basu, A., D. M. Goldenberg, et al. (1995). "The epithelial/carcinoma antigen EGP-1, recognized by monoclonal antibody RS7-3G11, is phosphorylated on serine 303." Int. J. Cancer 62: 472-479.
Brown, W. J. and M. G. Farquhar (1989). "Immunoperoxidase methods for the localization of antigens in cultured cells and tissue sections by electron microscopy." Methods Cell Biol 31: 553-69.
Brummelkamp, T. R., R. Bernards, et al. (2002). "A System for Stable Expression of Short Interfering RNAs in Mammalian Cells." Science 296(5567): 550-553. Chalk, A. M., C. Wahlestedt, et al. (2004). "Improved and automated prediction of effective siRNA." Biochem Biophys Res Commun 319(1): 264-74.
Chong, J. M. and D. W. Speicher (2001). "Determination of Disulfide Bond Assignments and N-Glycosylation Sites of the Human Gastrointestinal Carcinoma Antigen GA733-2 (CO17-1A, EGP, KS1-4, KSA, and Ep-CAM)." J. Biol. Chem. 276(8): 5804-5813.
Ciccarelli, F., A. Acciarito, et al. (2000). "Large and diverse numbers of human diseases with HIKE mutations." Hum. Mol. Genet. 9: 1001-7.
Cubas, R., S. Zhang, et al. (2010). "Trop2 expression contributes to tumor pathogenesis by activating the ERK MAPK pathway." Mol Cancer 9(1): 253.
El Sewedy, T., M. Fornaro, et al. (1998). "Cloning of the murine Trop2 gene: conservation of a PIP2-binding sequence in the cytoplasmic domain of Trop-2." Int J Cancer 75(2): 324-30.
Elbashir, S. M., J. Harborth, et al. (2001). "Duplexes of 21-nucleotide RNAs mediate RNA interference in cultured mammalian cells." Nature 411(6836): 494-8.
Fornaro, M., R. Dell'Arciprete, et al. (1995). "Cloning of the gene encoding TROP-2, a cell-surface glycoprotein expressed by human carcinomas." Int. J. Cancer 62: 610-8.
Freeley, M., J. Park, et al. (2007). "Loss of PTEN expression does not contribute to PDK-1 activity and PKC activation-loop phosphorylation in Jurkat leukaemic T cells." Cell Signal 19(12): 2444-57.
Giulietti, A., L. Overbergh, et al. (2001). "An overview of real-time quantitative PCR: applications to quantify cytokine gene expression." Methods 25(4): 386-401.
Goldstein, A. S., T. Stoyanova, et al. (2010). "Primitive origins of prostate cancer: In vivo evidence for prostate-regenerating cells and prostate cancer-initiating cells." Mol Oncol.
Guerra, E., M. Trerotola, et al. (2013). "The Trop-2 signalling network in cancer growth." Oncogene doi: 10.1038/onc.2012.151**. [Epub ahead of print].**
Guerra, E., M. Trerotola, et al. (2008). "A bi-cistronic CYCLIN D1-TROP2 mRNA chimera demonstrates a novel oncogenic mechanism in human cancer." Cancer Res 68(19): 8113-8121.
Hakomori, S. I. (2002). "Inaugural Article: The glycosynapse." Proc Natl Acad Sci U S A 99(1): 225-32.
Hemler, M. E. (2003). "Tetraspanin proteins mediate cellular penetration, invasion, and fusion events and define a novel type of membrane microdomain." Annu Rev Cell Dev Biol 19: 397-422.
Jacinto, E., V. Facchinetti, et al. (2006). "SIN1/MIP1 maintains rictor-mTOR complex integrity and regulates Akt phosphorylation and substrate specificity." Cell 127(1): 125-37.
Klein, C. E., B. Hartmann, et al. (1990). "Expression of 38-kD cell-surface glycoprotein in transformed human keratinocyte cell lines, basal cell carcinomas, and epithelial germs." J. Invest. Dermatol. 95: 74-82.
Koul, D., R. Shen, et al. (2005). "Targeting integrin-linked kinase inhibits Akt signaling pathways and decreases tumor progression of human glioblastoma." Mol Cancer Ther 4(11): 1681-8.
Le Naour, F., M. Andre, et al. (2006). "Membrane microdomains and proteomics: lessons from tetraspanin microdomains and comparison with lipid rafts." Proteomics 6(24): 6447-54.
Levy, S. and T. Shoham (2005). "The tetraspanin web modulates immune-signalling complexes." Nat Rev Immunol 5(2): 136-48.
Linnenbach, A. J., B. A. Seng, et al. (1993). "Retroposition in a family of carcinoma-associated antigen genes." Mol. Cell. Biol. 13: 1507-1515.
Livak, K. J. and T. D. Schmittgen (2001). "Analysis of relative gene expression data using real-time quantitative PCR and the 2(-Delta Delta C(T)) Method." Methods 25(4): 402-8.
Minguez, P., S. Gotz, et al. (2009). "SNOW, a web-based tool for the statistical analysis of protein-protein interaction networks." Nucleic Acids Res 37(Web Server issue): W109-14.
Naquet, P., H. Lepesant, et al. (1989). "Establishment and characterization of mouse thymic epithelial cell lines." Thymus 13(3-4): 217-26.
Parekh, D. B., W. Ziegler, et al. (2000). "Multiple pathways control protein kinase C phosphorylation." EMBO J 19(4): 496-503.
Polishchuk, R. S., E. V. Polishchuk, et al. (2000). "Correlative light-electron microscopy reveals the saccular-tabular ultrastructure of carriers operating between the Golgi apparatus and the plasma membrane." J. Cell Biol. 148: 45-58.
Raji, R., Guzzo, F., et al. (2011). "Uterine and ovarian carcinosarcomas overexpressing Trop -2 are sensitive to hRS7, a humanized anti-Trop-2 antibody." J Exp Clin Cancer Res 30(1): 106.
Rosse, C., M. Linch, et al. (2010). "PKC and the control of localized signal dynamics." Nat Rev Mol Cell Biol 11(2): 103-12.
Rossi, C., A. Di Lena, et al. (2008). "Intestinal tumour chemoprevention with the antioxidant lipoic acid stimulates the growth of breast cancer." Eur J Cancer 44: 2696 -2704.
Sarbassov, D. D., D. A. Guertin, et al. (2005). "Phosphorylation and regulation of Akt/PKB by the rictor-mTOR complex." Science 307(5712): 1098-101.
Semizarov, D., L. Frost, et al. (2003). "Specificity of short interfering RNA determined through gene expression signatures." Proc Natl Acad Sci U S A 100(11): 6347-52.
Trerotola, M., P. Cantanelli, et al. (2013). "Up-regulation of Trop-2 quantitatively stimulates human cancer growth." Oncogene 32 222-233.
Trerotola, M., S. Rathore, et al. (2010). "CD133, Trop-2 and alpha2beta1 integrin surface receptors as markers of putative human prostate cancer stem cells." Am J Transl Res 2(2): 135-44.
Varughese, J., Cocco, E., et al.(2011a). " Uterine serous papillary carcinomas overexpress human trophoblast-cell-surface marker (Trop-2) and are highly sensitive to immunotherapy with hRS7, a humanized anti-Trop-2 monoclonal antibody." Cancer 117(14): 3163-72,
Varughese, J., Cocco, E., et al.(2011b). " Cervical carcinomas overexpress human trophoblast cell-surface marker (Trop-2) and are highly sensitive to immunotherapy with hRS7, a humanized monoclonal anti-Trop-2 antibody." Am. J. Obstet. Gynecol. 205(6): 567.e1-7.
Varughese, J., Cocco, E., et al.(2011c). " High-grade, chemotherapy-resistant primary ovarian carcinoma cell lines overexpress human trophoblast cell-surface marker (Trop-2) and are highly sensitive to immunotherapy with hRS7, a humanized monoclonal anti-Trop -2 antibody." Gynecol Oncol. 122 (1): 171-7-
Wang, J., R. Day, et al. (2008). "Identification of Trop-2 as an oncogene and an attractive therapeutic target in colon cancers." Mol Cancer Ther 7(2): 280-5.
Wen, H. C., W. C. Huang, et al. (2003). "Negative regulation of phosphatidylinositol 3-kinase and Akt signalling pathway by PKC." Cell Signal 15(1): 37-45.
Zhang, X. A., A. L. Bontrager, et al. (2001). "Transmembrane-4 superfamily proteins associate with activated protein kinase C (PKC) and link PKC to specific beta(1) integrins." J Biol Chem 276(27): 25005-13.

## Claims

1. Method to predict *in vitro* the outcome of an anticancer therapy with drugs inhibiting the activity of a component of the Trop-2 signaling network, wherein said component is Akt, said method comprising or consisting of determining the expression levels of Trop-2 protein or of the corresponding mRNA in a biological sample before the treatment, an effective outcome occurring when an increase of the expression levels of Trop-2 protein or of the corresponding mRNA as compared to the levels in the corresponding normal tissues is detected.

2. Method according to claim 1, wherein the increase in the mRNA of Trop-2 in the tumor tissues compared to the corresponding normal tissues is equal to or greater than 10%, as quantified by means of RT-PCR or real time quantitative RT-PCR.

3. Method according to claim 1, wherein the increase in the Trop-2 protein in the tumor tissues compared to the corresponding normal tissues is equal to or greater than 10%, as quantified by means of immunohistochemistry, ELISA assays, Western blotting.

4. Method for *in vitro* screening of candidate drugs for the treatment or prevention of cancer, or the inhibition of cancer cell growth, wherein such drugs are directed against a component of the Trop-2 signaling network, wherein said component is Akt, said method comprising or consisting of the following steps:
a. administering the compound to be tested, in sterile saline solution, to cells expressing Trop-2 and not expressing Trop-2, wherein the non-expressing cells act as a control of specificity; in parallel administering the sterile saline solution alone to cells expressing Trop-2 and not expressing Trop-2 (untreated controls), wherein the sterile saline solution alone acts as a control of activity;
b. detecting the biological activity of the compound of step (a) on cells expressing Trop-2 in comparison with treated controls not expressing Trop-2 and untreated controls expressing and not expressing Trop-2, in which the biological activity is a reduction of cell proliferation in cells expressing Trop-2 compared to treated control cells not expressing Trop-2 and compared to untreated control cells expressing and not expressing Trop-2;
c. selecting the compound of step (a) that reduces cell proliferation by at least 10% in treated cells expressing Trop-2, in comparison with cell proliferation in treated cells not expressing Trop-2 and in untreated cells expressing and not expressing Trop-2.

5. Compound inhibiting the activity of a component of the Trop-2 signaling network, wherein said component is Akt, for use in the treatment of tumours expressing levels of Trop-2 protein or corresponding mRNA higher than normal tissue wherein said compound is chosen from the group consisting of MK-2206, A6730, Perifosine, GSK690693, GSK2110183, GDC-0068, AT7867, ARQ092, AZD5363, A-674563, PHT-427, PF-04691502, SureCN1078972, 842148-40-7, AC1NX3D3, MLS002702033, SureCN10005574, SureCN1559590, SureCN570829, SH-5, SH-6, Honokiol, Miltefosine, Triciribine phosphate, monoclonal antibodies and antisense oligonucleotides.

6. Compound according to claim 5 for use according to claim 5, in combination with drugs chosen from the group consisting of chemotherapic drugs, alkylating agents, antimetabolites, anti-tumor antibiotics, topoisomerase inhibitors, mitotic inhibitors, corticosteroids, differentiating agents, hormone therapy, targeted kinase inhibitors, the proteosome inhibitor bortezomib.

7. Method for the *in vivo* screening of candidate drugs for the treatment or prevention of cancer, or the inhibition of cancer cell growth, wherein such drugs are directed against a component of the signalling network of Trop-2, wherein said component is Akt, said method comprising the steps of:
a. administering the compound to be tested, directed against a component of the signalling network of Trop-2, wherein said component is Akt, in sterile saline solution, to tumors which are positive or negative for the Trop-2 biological marker according to claim no. 2, in pre-clinical models, excluding humans, where the negative tumors act as a control of specificity; in parallel administering the sterile saline solution alone to tumors which are positive or negative for the Trop-2 biological marker (untreated controls), where the sterile saline solution alone acts as a control of activity;
b. detecting the biological activity of the compound of step (a) on tumors which are positive for the Trop-2 biological marker, in comparison with treated controls negative for the Trop-2 biological marker and untreated controls positive and negative for the Trop-2 biological marker, where the biological activity is a reduction of tumor growth in tumors positive for the Trop-2 biological marker compared to control tumors negative for the Trop-2 biological marker and compared to untreated control tumors positive and negative for the Trop-2 biological marker;
c. selecting the compound of step (a) that reduces tumor growth by at least 10% in tumors which are positive for the Trop-2 biological marker, in comparison with treated control tumors negative for the Trop-2 biological marker and untreated control tumors positive and negative for the Trop-2 biological marker.

## Patentansprüche

1. Methode, um *in vitro* das Ergebnis einer Anti-Krebs-Therapie mit Arzneimitteln vorherzusagen, die die Aktivität eines Bestandteils des Trop-2-Signalisierungsnetzes hemmen, wobei besagter Bestandteil Akt ist und die Methode die Bestimmung der Expressionswerte des Trop-2-Proteins oder der entsprechenden mRNA in einer biologischen Probe vor der Behandlung umfasst oder daraus besteht, wobei ein wirksames Ergebnis eintritt, wenn eine Erhöhung der Expressionswerte des Trop-2-Proteins oder der entsprechenden mRNA im Vergleich zu den Werten in den entsprechenden normalen Geweben nachgewiesen wird.

2. Methode nach Anspruch 1, wobei die Erhöhung von Trop-2 im mRNA in den Tumorgeweben im Vergleich zu den entsprechenden normalen Geweben gleich oder größer als 10% ist, wie anhand von RT-PCR oder mengenmäßiger RT-PCR in Echtzeit quantifiziert wird.

3. Methode nach Anspruch 1, wobei die Erhöhung des Trop-2-Proteins im Tumorgewebe im Vergleich zu den entsprechenden normalen Geweben gleich oder größer als 10% ist, wie anhand der Immunhistochemie, ELISA, Western Blotting quantifiziert wird.

4. Methode für das *In vitro*-Screening von Medikamentenkandidaten für die Behandlung oder Vorbeugung von Krebs oder die Hemmung des Wachstums der Krebszellen, wobei solche Arzneimittel gegen einen Bestandteil des Trop-2-Signalisierungsnetzes gerichtet sind, wobei besagter Bestandteil Akt ist und besagte Methode die folgenden Schritte umfasst und aus ihnen besteht:
a. Verabreichung der zu testenden Verbindung in steriler Kochsalzlösung an Zellen, die Trop-2 exprimieren und Trop-2 nicht exprimieren, wobei die nicht exprimierenden Zellen als Kontrolle der Spezifität wirken; parallel dazu wird die sterile Kochsalzlösung Zellen allein verabreicht, die Trop-2 exprimieren und Trop-2 nicht exprimieren (unbehandelte Kontrollen), wobei die sterile Kochsalzlösung allein als Kontrolle der Aktivität dient;
b. Nachweis der biologischen Aktivität der Verbindung von Schritt (a) an Zellen, die Trop-2 exprimieren, im Vergleich zu behandelten Kontrollen, die Trop-2 nicht exprimieren, und zu unbehandelten Kontrollen, die Trop-2 exprimieren und nicht exprimieren, wobei die biologische Aktivität eine Verringerung der Zellproliferation in Zellen ist, die Trop-2 exprimieren, im Vergleich zu behandelten Kontrollzellen, die Trop-2 nicht exprimieren, und zu unbehandelten Kontrollzellen, die Trop-2 exprimieren und nicht exprimieren;
c. Auswahl der Verbindung aus Schritt (a), die die Zellproliferation in behandelten Zellen, die Trop-2 exprimieren, um mindestens 35 % verringert, im Vergleich zur Zellproliferation in behandelten Zellen, die Trop-2 nicht exprimieren, und in unbehandelten Zellen, die Trop-2 exprimieren und nicht exprimieren.

5. Verbindung, die die Aktivität eines Bestandteils des Trop-2-Signalisierungsnetzes hemmt, wobei der Bestandteil Akt ist, zur Verwendung bei der Behandlung von Tumoren, die höhere Werte an Trop-2-Protein oder entsprechenden mRNA als normales Gewebe exprimieren, wobei die Verbindung ausgewählt ist aus der Gruppe bestehend aus MK- 2206, A6730, Perifosin, GSK690693, GSK2110183, GDC-0068, AT7867, ARQ092, AZD5363, A-674563, PHT-427, PF-04691502, SureCN1078972, 842148-40-7, AC1NX3D3, MLS002702033, SureCN 10005574, SureCN 1559590, 10SureCN570829, SH-5, SH-6, Honokiol, Miltefosin, Triciribinphosphat, monoklonale Antikörpern und Antisense-Oligonukleotiden.

6. Verbindung nach Anspruch 5 zur Verwendung nach Anspruch 5 in Kombination mit Arzneimitteln ausgewählt aus der Gruppe bestehend aus Chemotherapeutika, Alkylierungsmitteln, Antimetaboliten, Anti-Tumor-Antibiotika, Topoisomerase-Hemmern, Mitosehemmern, Kortikosteroiden, differenzierenden Wirkstoffen, Hormontherapie, gezielten Kinase-Hemmern, dem Proteosomen-Hemmer Bortezomib.

7. Methode für das *In vivo*-Screening von Arzneimittelkandidaten zur Behandlung oder Vorbeugung von Krebs oder zur Hemmung des Wachstums von Krebszellen, wobei diese Arzneimittel gegen einen Bestandteil des Signalisierungsnetzes von Trop-2 gerichtet sind, worin besagter Bestandteil Akt ist und die Methode die folgenden Schritte umfasst:
a. Verabreichung der zu testenden Verbindung, gerichtet gegen einen Bestandteil des Signalisierungsnetzes von Trop-2, wobei der Bestandteil Akt ist, in steriler Kochsalzlösung an Tumoren, die für den biologischen Trop-2-Marker nach Anspruch 2 positiv oder negativ sind, in präklinischen Modellen, ausgenommen Menschen, in denen die negativen Tumore als Kontrolle der Spezifität wirken; parallel dazu wird die sterile Kochsalzlösung allein Tumoren verabreicht, die für den biologischen Trop-2-Marker positiv oder negativ sind (unbehandelte Kontrollen), in welchem die sterile Kochsalzlösung allein als Kontrolle der Aktivität dient;
b. Nachweis der biologischen Aktivität der Verbindung aus Schritt (a) an Tumoren, die für den biologischen Trop-2-Marker positiv sind, im Vergleich zu behandelten Kontrollen, die negativ für den biologischen Trop-2-Marker sind und zu unbehandelten Kontrollen positiv und negativ für den biologischen Trop-2-Marker, in welchem die biologische Aktivität eine Verringerung des Tumorwachstums in Tumoren ist, die positiv für den biologischen Trop-2-Marker im Vergleich zu Kontrolltumoren negativ für den biologischen Trop-2-Marker und im Vergleich zu unbehandelten Kontrolltumoren positiv und negativ für den biologischen Trop-2-Marker ist;
c. Auswahl der Verbindung von Schritt (a), die das Tumorwachstum um mindestens 10% bei Tumoren verringert, die positiv für den biologischen Trop-2-Marker sind, im Vergleich zu behandelten Kontrolltumoren negativ für den biologischen Trop-2-Marker und zu unbehandelten Kontrolltumoren positiv und negativ für den biologischen Trop-2-Marker sind.

## Revendications

1. Procédé permettant de prédire *in vitro* le résultat d'une thérapie anticancéreuse à l'aide de médicaments inhibant l'activité d'un composant du réseau de signalisation de Trop-2, dans lequel ce composant est Akt, ledit procédé comprenant ou consistant dans la détermination des niveaux d'expression de la protéine Trop-2 ou de l'ARNm correspondant dans un échantillon biologique avant le traitement, un résultat tangible se produisant lorsqu'une augmentation des niveaux d'expression de la protéine Trop-2 ou de l'ARNm correspondant, par comparaison avec les niveaux existant dans les tissus normaux correspondants, est détectée.

2. Procédé selon la revendication 1, dans lequel l'augmentation de l'ARNm de Trop-2 dans les tissus tumoraux, par comparaison aux tissus normaux correspondants, est supérieure ou égale à 10%, telle que quantifiée au moyen d'une RT-PCR ou d'une RT-PCR quantitative en temps réel.

3. Procédé selon la revendication 1, dans lequel l'augmentation de la protéine Trop-2 dans les tissus tumoraux, par comparaison aux tissus normaux correspondants, est supérieure ou égale à 10%, telle que quantifiée au moyen d'une immunohistochimie, de dosages ELISA, d'un buvardage de Western.

4. Procédé permettant le criblage *in vitro* de candidats médicaments destinés au traitement ou à la prévention du cancer, ou encore l'inhibition de la croissance de cellules cancéreuses, dans lequel ces médicaments sont dirigés contre un composant du réseau de signalisation de Trop-2, dans lequel ledit composant est Akt, ce procédé comportant ou consistant dans les étapes suivantes:
a. administration du composé à tester, dans une solution saline stérile, à des cellules exprimant Trop-2 et n'exprimant pas Trop-2, sachant que les cellules non exprimantes jouent un rôle de contrôle de spécificité ; parallèlement, administration uniquement de la solution saline stérile à des cellules exprimant Trop-2 et n'exprimant pas Trop-2 (témoins non traités), sachant que la solution saline stérile seule joue un rôle de contrôle d'activité;
b. détection de l'activité biologique du composé de l'étape (a) sur des cellules exprimant Trop-2 par comparaison aux témoins traités n'exprimant pas Trop-2 et aux témoins non traités exprimant et n'exprimant pas Trop-2, dans laquelle l'activité biologique est une réduction de la prolifération cellulaire dans des cellules exprimant Trop-2 comparées aux cellules témoins traitées n'exprimant pas Trop-2 et comparées aux cellules témoins non traitées exprimant et n'exprimant pas Trop-2;
c. sélection du composé de l'étape (a) qui réduit la prolifération cellulaire d'au moins 10% dans les cellules traitées exprimant Trop-2, par comparaison avec la prolifération cellulaire dans les cellules traitées n'exprimant pas Trop-2 et dans les cellules non traitées exprimant et n'exprimant pas Trop-2.

5. Composé inhibant l'activité d'un composant du réseau de signalisation de Trop-2, dans lequel ledit composant est Akt, destiné à être utilisé dans le traitement des tumeurs exprimant des niveaux de protéine Trop-2 ou d'ARNm correspondant plus élevés que ceux des tissus normaux, et dans lequel ce composant est choisi dans le groupe constitué par le MK-2206, l'A6730, la périfosine, le GSK690693, le GSK2110183, le GDC-0068, l'AT7867, l'ARQ092, l'AZD5363, l'A-674563, le PHT-427, le PF-04691502, le SureCN1078972, le 842148-40-7, l'AC1NX3D3, le MLS002702033, le SureCN10005574, le SureCN1559590, le SureCN570829, le SH-5, le SH-6, l'honokiol, la miltéfosine, le phosphate de triciribine, les anticorps monoclonaux et les oligonucléotides antisens.

6. Composé selon la revendication 5 destiné à être utilisé selon la revendication 5, en association avec des médicaments choisis dans le groupe constitué par les médicaments chimiothérapeutiques, les agents alkylants, les antimétabolites, les antibiotiques antitumoraux, les inhibiteurs de topoisomérase, les inhibiteurs mitotiques, les corticoïdes, les agents de différenciation, l'hormonothérapie, les inhibiteurs de kinase ciblés, l'inhibiteur du protéasome bortézomib.

7. Procédé permettant le criblage *in vivo* de candidats médicaments destinés au traitement ou à la prévention du cancer, ou encore l'inhibition de la croissance de cellules cancéreuses, dans lequel ces médicaments sont dirigés contre un composant du réseau de signalisation de Trop-2, dans lequel ce composant est Akt, ce procédé comportant les étapes suivantes:
a. administration du composé à tester, dirigé contre un composant du réseau de signalisation Trop-2, sachant que ledit composant est Akt, dans une solution saline stérile, aux tumeurs qui s'avèrent positives ou négatives pour le marqueur biologique Trop-2 selon la revendication 2, sur des modèles précliniques à l'exclusion des humains, sachant que les tumeurs négative jouent un rôle de contrôle de spécificité; parallèlement, administration uniquement de la solution saline stérile aux tumeurs qui s'avèrent positives ou négatives pour le marqueur biologique Trop-2 (témoins non traités), sachant que la solution saline stérile seule joue un rôle de contrôle d'activité;
b. détection de l'activité biologique du composé de l'étape (a) sur les tumeurs qui s'avèrent positives pour le marqueur biologique Trop-2, par comparaison aux témoins traités négatifs pour le marqueur biologique Trop-2 et aux témoins non traités positifs et négatifs pour le marqueur biologique Trop-2, dans laquelle l'activité biologique est une réduction de la croissance tumorale des tumeurs positives pour le marqueur biologique Trop-2 comparées au tumeurs témoins négatives pour le marqueur biologique Trop-2 et comparées aux tumeurs témoins non traitées positives et négatives pour le marqueur biologique Trop-2;
c. sélection du composé de l'étape (a) qui réduit la croissance tumorale d'au moins 10% dans les tumeurs qui s'avèrent positives pour le marqueur biologique Trop-2, par comparaison avec les tumeurs témoins traitées négatives pour le marqueur biologique Trop-2 et les tumeurs témoins non traitées positives et négatives pour le marqueur biologique Trop-2.


### SEQUENCE LISTING

<110> Alberti, saverio
<120> Use of Trop-2 as predictive marker of response to anti-tumor therapy based on inhibitors of CD9, Akt and molecules of the tetraspanin signalling network
<140> ITPCT29144
<160> 36
<170> BiSSAP 1.2
<210> 1
   <211> 19
   <212> DNA
   <213> Mus musculus
<220>
   <221> source
   <222> 1..19
   <223> /mol_type="unassigned DNA" /note='target sequence for CD9 silencing, forward" /organism= Mus musculus"
<400> 1
   ggtagcaagt gcatcaaat 19
<210> 2
   <211> 19
   <212> DNA
   <213> Mus musculus
<220>
   <221> source
   <222> 1..19
   <223> /mol_type="unassigned DNA" /note= "target sequence for CD9 silencing, reverse" /organism= Mus musculus"
<400> 2
   atttgatgca cttgctacc 19
<210> 3
   <211> 64
   <212> DNA
   <213> Artificial sequence
<220>
   <221> source
   <222> 1..64
   <223> /mol_type="unassigned DNA" /note="oligonucleotide for the transcription of hairpin RNA (shRNa)" /organism="Artificial Sequence"
<400> 3
   gatccccggt agcaagtgca tcaaatttca agagaatttg atgcacttgc tacctttttg 60
   gaaa 64
<210> 4
   <211> 64
   <212> DNA
   <213> Artificial sequence
<220>
   <221> source
   <222> 1..64
   <223> /mol_type="unassigned DNA"
   /note='oligonucleotide for the transcription of hairpin RNA (shRNA)"
   /organism="Artificial sequence"
<400> 4
   agcttttcca aaaaggtagc aagtgcatca aattctcttg aaatttgatg cacttgctac 60
cggg 64
<210> 5
   <211> 19
   <212> DNA
   <213> Mus musculus
<220>
   <221> source
   <222> 1..19
   <223> /mol_type="unassigned DNA" /organism="Mus musculus"
<400> 5
   gcacctttat tggctacaa 19
<210> 6
   <211> 19
   <212> DNA
   <213> Mus musculus
<220>
   <221> source
   <222> 1..19
   <223> /mol_type="unassigned DNA" /note= "target sequence #1 for AKT silencing, reverse" /organism= "Mus musculus"
<400> 6
   ttgtagccaa taaaggtgc 19
<210> 7
   <211> 60
   <212> DNA
   <213> Artificial sequence
<220>
   <221> source
   <222> 1..60
   <223> /mol_type="unassigned DNA" /note="oligonucleotide for the transcription of hairpin RNA (shRNA)" /organism="Artificial sequence"
<400> 7
   gatccccgca cctttattgg ctacaattca agagattgta gccaataaag gtgctttttc 60
<210> 8
   <211> 60
   <212> DNA
   <213> Artificial Sequence
<220>
   <221> source
   <222> 1..60
   <223> /mol_type="unassigned DNA" /note="oligonucleotide for the transcription of hairpin RNA (shRNA)" /organi sm="Artificial sequence"
<400> 8
   tcgagaaaaa gcacctttat tggctacaat ctcttgaatt gtagccaata aaggtgcggg 60
<210> 9
   <211> 19
   <212> DNA
   <213> Mus musculus
<220>
   <221> source
   <222> 1..19
   <223> /mol_type="unassigned DNA" /organism="Mus musculus"
<400> 9
   ggaaggtgat tctggtgaa 19
<210> 10
   <211> 19
   <212> DNA
   <213> Mus musculus
<220>
   <221> source
   <222> 1..19
   <223> /mol_type="unassigned DNA" /organism="Mus musculus"
<400> 10
   ttcaccagaa tcaccttcc 19
<210> 11
   <211> 60
   <212> DNA
   <213> Artificial sequence
<220>
   <221> source
   <222> 1..60
   <223> /mol_type="unassigned DNA" /note="oligonucleotide for the transcription of hairpin RNA (shRNA)" /organism="Artificial sequence"
<400> 11
   gatccccgga aggtgattct ggtgaattca agagattcac cagaatcacc ttcctttttc 60
<210> 12
   <211> 60
   <212> DNA
   <213> Artificial sequence
<220>
   <221> source
   <222> 1..60
   <223> /mol_type="unassigned DNA" /note="oligonucleotide for the transcription of hairpin RNA (shRNA)" /organism="Artificial sequence"
<400> 12
   tcgagaaaaa ggaaggtgat tctggtgaat ctcttgaatt caccagaatc accttccggg 60
<210> 13
   <211> 27
   <212> DNA
   <213> Artificial sequence
<220>
   <221> source
   <222> 1..27
   <223> /mol-type="unassigned DNA" /note="PCR primer for human TROP2, forward" /organism="Artificial sequence"
<400> 13
   gcgattctcg agtccggtcc gcgttcc 27
<210> 14
   <211> 27
   <212> DNA
   <213> Artificial sequence
<220>
   <221> source
   <222> 1..27
   <223> /mol_type="unassigned DNA" /note="PCR primer for human TROP2, reverse" /organism="Artificial sequence"
<400> 14
   gcgccggtac caagctcggt tcctttc 27
<210> 15
   <211> 19
   <212> DNA
   <213> Mus musculus
<220>
   <221> source
   <222> 1..19
   <223> /mol_type="unassigned DNA" /organism="Mus musculus"
<400> 15
   ctttcaaacc tgagtataa 19
<210> 16
   <211> 19
   <212> DNA
   <213> Mus musculus
<220>
   <221> source
   <222> 1..19
   <223> /mol_type="unassigned DNA" /organism="Mus musculus"
<400> 16
   ttatactcag gtttgaaag 19
<210> 17
   <211> 64
   <212> DNA
   <213> Artificial sequence
<220>
   <221> source
   <222> 1..64
   <223> /mol_type="unassigned DNA" /note="oligonucleotide for the transcription of hairpin RNA (shRNA)" /organism="Artificial sequence"
<400> 17
   gatccccctt tcaaacctga gtataattca agagattata ctcaggtttg aaagtttttg 60
   gaaa 64
<210> 18
   <211> 64
   <212> DNA
   <213> Artificial sequence
<220>
   <221> source
   <222> 1..64
   <223> /mol_type="unassigned DNA" /note="oligonucleotide for the transcription of hairpin RNA (shRNA)" /organism="Artificial sequence"
<400> 18
   agcttttcca aaaactttca aacctgagta taatctcttg aattatactc aggtttgaaa 60
   gggg 64
<210> 19
   <211> 19
   <212> DNA
   <213> Homo sapiens
<220>
   <221> source
   <222> 1..19
   <223> /mol_type="unassigned DNA" /organism="Homo sapiens"
<400> 19
   cttgacaacg ttaataacg 19
<210> 20
   <211> 19
   <212> DNA
   <213> Homo sapiens
<220>
   <221> source
   <222> 1..19
   <223> /mol_type="unassigned DNA" /organism="Homo sapiens"
<400> 20
   cgttattaac gttgtcaag 19
<210> 21
   <211> 64
   <212> DNA
   <213> Artificial sequence
<220>
   <221> source
   <222> 1..64
   <223> /mol_type="unassigned DNA" /note='oligonucleotide for the transcription of hairpin RNA (shRNA)" /organism="Artificial Sequence"
<400> 21
   gatccccctt gacaacgtta ataacgttca agagacgtta ttaacgttgt caagtttttg 60
   gaaa 64
<210> 22
   <211> 64
   <212> DNA
   <213> Artificial sequence
<220>
   <221> source
   <222> 1..64
   <223> /mol_type="unassigned DNA" /note="oligonucleotide for the transcription of hairpin RNA (shRNA)" /organism="Artificial Sequence"
<400> 22
   agcttttcca aaaacttgac aacgttaata acgtctcttg aacgttatta acgttgtcaa 60
   gggg 64
<210> 23
   <211> 19
   <212> DNA
   <213> Homo sapiens
<220>
   <221> source
   <222> 1..19
   <223> /mol_type="unassigned DNA" /organism="Homo sapiens"
<400> 23
   tgcaggaagt ggtgggaat 19
<210> 24
   <211> 19
   <212> DNA
   <213> Homo sapiens
<220>
   <221> source
   <222> 1..19
   <223> /mol_type="unassigned DNA" /organism="Homo sapiens"
<400> 24
   attcccacca cttcctgca 19
<210> 25
   <211> 64
   <212> DNA
   <213> Artificial sequence
<220>
   <221> source
   <222> 1..64
   <223> /mol_type="unassigned DNA" /note='oligonucleotide for the transcription of hairpin RNA (shRNA)" /organism="Artificial sequence"
<400> 25
   gatcccctgc aggaagtggt gggaatttca agagaattcc caccacttcc tgcatttttg 60
   gaaa 64
<210> 26
   <211> 64
   <212> DNA
   <213> Artificial sequence
<220>
   <221> source
   <222> 1..64
   <223> /mol_type="unassigned DNA" /note="oligonucleotide for the transcription of hairpin RNA (shRNA)" /organism="Artificial sequence"
<400> 26
   agcttttcca aaaatgcagg aagtggtggg aattctcttg aaattcccac cacttcctgc 60
   aggg 64
<210> 27
   <211> 27
   <212> DNA
   <213> Artificial Sequence
<220>
   <221> source
   <222> 1..27
   <223> /mol_type="unassigned DNA" /note="PCR oligonucleotide for delta-cyto Trop-2 mutant" /organism="Artificial Sequence"
<400> 27
   ctcggatcca ccatggctcg gggcccc 27
<210> 28
   <211> 27
   <212> DNA
   <213> Artificial sequence
<220>
   <221> source
   <222> 1..27
   <223> /mol_type="unassigned DNA" /note="PCR oligonucleotide for delta-cyto Trop-2 mutant" /organism="Artificial Sequence"
<400> 28
   ctcgaattcc cggttggtga tcaccag 27
<210> 29
   <211> 27
   <212> DNA
   <213> Artificial Sequence
<220>
   <221> source
   <222> 1..27
   <223> /mol_type="unassigned DNA" /note="PCR oligonucleotide for E-K Trop-2 mutant" /organism="Artificial Sequence"
<400> 29
   gcggaattcc gtccggtccg cgttcct 27
<210> 30
   <211> 64
   <212> DNA
   <213> Artificial Sequence
<220>
   <221> source
   <222> 1..64
   <223> /mol_type="unassigned DNA" /note="PCR oligonucleotide for E-K Trop-2 mutant" /organism="Artificial Sequence"
<400> 30
   gcgtctagac tacaagctcg gtttctttct caacttcccc agtttcttga tcttcacctt 60
   cttg 64
<210> 31
   <211> 27
   <212> DNA
   <213> Artificial Sequence
<220>
   <221> source
   <222> 1..27
   <223> /mol_type="unassigned DNA" /note="PCR oligonucleotide for delta-HIKE Trop-2 mutant" /organism="Artificial Sequence"
<400> 31
   gcggaattcc gtccggtccg cgttcct 27
<210> 32
   <211> 75
   <212> DNA
   <213> Artificial Sequence
<220>
   <221> source
   <222> 1..75
   <223> /mol_type="unassigned DNA" /note="PCR oligonucleotide for delta-HIKE Trop-2 mutant" /organism="Artificial Sequence"
<400> 32
   gcgtctagac tacaagctcg gttcctttct caactccccc agttccttga tctccactct 60
   ccggttggtg atcac 75
<210> 33
   <211> 27
   <212> DNA
   <213> Artificial Sequence
<220>
   <221> source
   <222> 1..27
   <223> /mol_type="unassigned DNA" /note="PCR oligonucleotide for S303A Trop-2 mutant" /organism="Artificial Sequence"
<400> 33
   ctcggatcca ccatggctcg gggcccc 27
<210> 34
   <211> 85
   <212> DNA
   <213> Artificial Sequence
<220>
   <221> source
   <222> 1..85
   <223> /mol_type="unassigned DNA" /note="PCR oligonucleotide for S303A Trop-2 mutant" /organism="Artificial sequence"
<400> 34
   gcgtctagac tacaagctcg gttcctttct caactccccc agttccttga tctccacctt 60
   cttgtacttc cccgcctttc tccgg 85
<210> 35
   <211> 27
   <212> DNA
   <213> Artificial Sequence
<220>
   <221> source
   <222> 1..27
   <223> /mol_type="unassigned DNA" /note="PCR oligonucleotide for S322A Trop-2 mutant" /organism="Artificial Sequence"
<400> 35
   ctcggatcca ccatggctcg gggcccc 27
<210> 36
   <211> 39
   <212> DNA
   <213> Artificial Sequence
<220>
   <221> source
   <222> 1..39
   <223> /mol_type="unassigned DNA" /note="PCR oligonucleotide for S322A Trop-2 mutant" /organism="Artificial Sequence"
<400> 36
   gcgtctagac tacaaggccg gttcctttct caactcccc 39